# EUROPEAN PATENT APPLICATION

(11) **EP 2 113 568 A1**
(43) Date of publication of application: **04.11.2009**
(21) Application number: 08103781.4
(22) Date of filing: 30.04.2008
(51) Int. Cl.: C12N 15/85, A01K 67/027, C12N 5/10, C07K 14/525

(54) **Knock-in mouse for modelling blockade of human TNFalpha**

(71) Applicant: Deutsches Rheuma-Forschungszentrum Berlin, 10117 Berlin (DE)
(72) Inventor: Nedospasov, Sergei, 10178 Berlin (DE)
(74) Representative: Ziebig, Marlene

(57) **Abstract**

The invention relates to a transgenic mouse, wherein the complete endogenous murine TNFα-gene including its upstream regulatory sequences is replaced by the human homologous TNFα-gene including its upstream regulatory sequences or a mutant thereof, wherein the mutant is characterized by deletion, addition, substitution, and/or inversion of one or more nucleotides so that the functional properties of the modified human TNFα-gene are conserved and cells, tissues and cell lines derived thereof. The invention further relates to an in-vivo method of screening, investigating and/or preclinical testing of compounds and/or drug candidates for human anti-TNFα therapy using said transgenic mouse and an analogous method using the cells, tissues and cell lines derived thereof.

SEQUENCE LISTING

<110> Deutsches Rheuma-Forschungszentrum

<120> Knock-in mouse for modelling blockade of human TNFalpha

<130> P538508 EP

<160> 14

<170> PatentIn version 3.3

<210> 1
<211> 4534
<212> DNA
<213> mus musculus

<400> 1
tcagagtgaa aggagaaggc ttgtgaggtc cgtgaattcc cagggctgag ttcattccct 60

ctggggctgc cccatactca tcccattacc ccccccacca gccctcccaa agcccatgca 120

cacttcccaa ctctcaagct gctctgcctt cagccacttc ctccaagaac tcaaacaggg 180

ggctttccct cctcaatatc atgtctcccc ccttatgcac ccagctttca gaagcacccc 240

cccatgctaa gttctccccc atggatgtcc catttagaaa tcaaaaggaa atagacacag 300

gcatggtctt tctacaaaga aacagacaat gattagctct ggaggacaga gaagaaatgg 360

gtttcagttc tcagggtcct atacaacaca cacacacaca cacacacaca cacacacaca 420

cacacaccct cctgattggc cccagattgc cacagaatcc tggtggggac gacgggggag 480

agattccttg atgcctgggt gtccccaact ttccaaaccc tctgcccccg cgatggagaa 540

gaaaccgaga cagaggtgta gggccactac cgcttcctcc acatgagatc atggttttct 600

ccaccaagga agttttccga gggttgaatg agagcttttc cccgccctct tccccaaggg 660

ctataaaggc ggccgtctgc acagccagcc agcagaagct ccctcagcga ggacagcaag 720

ggactagcca ggagggagaa cagaaactcc agaacatctt ggaaatagct cccagaaaag 780

caagcagcca accaggcagg ttctgtccct ttcactcact ggcccaaggc gccacatctc 840

cctccagaaa agacaccatg agcacagaaa gcatgatccg cgacgtggaa ctggcagaag 900

aggcactccc ccaaaagatg gggggcttcc agaactccag gcggtgccta tgtctcagcc 960

tcttctcatt cctgcttgtg gcaggggcca ccacgctctt ctgtctactg aacttcgggg 1020

tgatcggtcc ccaaagggat gaggtgagtg tctgggcaac ccttattctc gctcacaagc 1080

aaaacgggtt aggagggcaa gaaggacagt gtgagggaaa gaagtgggct aatgggcagg 1140

gcaaggtgga ggagagtgtg gaggggacag agtcaggacc tcggacccat gcgtccagct 1200

gactaaacat ccttcgtcgg atgcacagag agatgaatga acgaacaagt gtgttcacac 1260

gtggagagat ctggaaagat gtggccaggg gaagagggga taagcaagag ataaaactca 1320

gagacagaaa tgagagaggc atgagagata aggaggaaga tgaaggggag ataacgggag 1380

atcaagcaca gagggcaccg cagaaagaag ccgtgggttg gacagatgaa tgaatgaaga 1440

agaaaacaca aagtgggggg tgggtggggc aaagaggaac tgtaagcggg gcaatcagcc 1500

gggagcttct cctttggggt gagtctgtct taactaacct ccttttccta cacagaagtt 1560
cccaaatggc ctccctctca tcagttctat ggcccagacc ctcacactca gtaagtgttc 1620
ccacacctct ctcttaattt aagatggaga agggcagtta ggcatgggat gagatggggt 1680
ggggggaaaa cttaaagctt tggtttggga ggaaaggggt ctaagtgcat agatgcttgc 1740
tgggaagcct aaaaggctca tccttgcctt tgtctcttcc cctccaggat catcttctca 1800
aaattcgagt gacaagcctg tagcccacgt cgtaggtaag atttctttac atgtgccttg 1860
agaatgaagg ggcatgattt tggggggcgg gttgaggggt gtcgagccag gctgagaaaa 1920
gacagagctc ttagagacag cacgtgagag tcagagcagt gactcaaaag caaggcatca 1980
gggggccacc cgggacctca tagccaatgg gatgtggaaa gacagagggt gcaggaaccg 2040
gaagtgaagt gtgggtagct gctgaggctc aggatgtgga gtgtgaacta agagggtgac 2100
actgactcaa tcctcccccc ccccctcagc aaaccaccaa gtggaggagc agctggagtg 2160
gctgagccag cgcgccaacg ccctcctggc caacggcatg gatctcaaag acaaccaact 2220
agtggtgcca gccgatgggt tgtaccttgt ctactcccag gttctcttca agggacaagg 2280
ctgccccgac tacgtgctcc tcacccacac cgtcagccga tttgctatct cataccagga 2340
gaaagtcaac ctcctctctg ccgtcaagag cccctgcccc aaggacaccc ctgagggggc 2400
tgagctcaaa ccctggtatg agcccatata cctgggagga gtcttccagc tggagaaggg 2460
ggaccaactc agcgctgagg tcaatctgcc caagtactta gactttgcgg agtccgggca 2520
ggtctacttt ggagtcattg ctctgtgaag ggaatgggtg ttcatccatt ctctacccag 2580
cccccactct gaccccttta ctctgacccc tttattgtct actcctcaga gcccccagtc 2640
tgtgtccttc taacttagaa aggggattat ggctcagagt ccaactctgt gctcagagct 2700
ttcaacaact actcagaaac acaagatgct gggacagtga cctggactgt gggcctctca 2760
tgcaccacca tcaaggactc aaatgggctt tccgaattca ctggagcctc gaatgtccat 2820
tcctgagttc tgcaaaggga gagtggtcag gttgcctctg tctcagaatg aggctggata 2880
agatctcagg ccttcctacc ttcagacctt tccagactct tccctgaggt gcaatgcaca 2940
gccttcctca cagagccagc ccccctctat ttatatttgc acttattatt tattatttat 3000
ttattattta tttatttgct tatgaatgta tttatttgga aggccggggt gtcctggagg 3060
acccagtgtg ggaagctgtc ttcagacaga catgttttct gtgaaaacgg agctgagctg 3120
tccccacctg gcctctctac cttgttgcct cctcttttgc ttatgtttaa aacaaaatat 3180
ttatctaacc caattgtctt aataacgctg atttggtgac caggctgtcg ctacatcact 3240
gaacctctgc tccccacggg agccgtgact gtaattgccc tacagtcaat tgagagaaat 3300
aaagatcgct tggaaaagaa atgtgatttc tgtcttggga tgaagtctgc atccatctct 3360
ttgcggaggc ctaaagtctc tgggtccaga tctcagtctt tatacccctg ggccattaag 3420
acccccaaga cccccgtgga acaaaaggca gccaacatcc ctacctctcc cccggaaaca 3480
ggagcctaac cctaattacc tttgccctgg ggcatgggaa tttcccactc tgggaattcc 3540

aatacctgct gggaaagtac tgcagttctt tccggctgtt gtggctccca tggagctggg 3600

gaggagccac attctcaggc acctgaatca cagccaagga agagccgcag agcttcaggt 3660

gtctaactgc gcaccctgca ccattcaacc tgcctctgaa cagacagagc gggcatggtc 3720

cacggagagg agacctaagc tctatccctg ctccatgctg ggctgggctc agccagctct 3780

gcctttcggt caccagcccc tttatttttc caaacctctg aagatgagtg aaatagtttt 3840

taaaaagaca cagggctgaa aatgtgactc tgtggagcat gttggggtga gggtggatgc 3900

tcagtgtatg ggataccctg aattcaaaac ccaaagcaac acaacaaaga aggagccatt 3960

gctggctttg gttttccagt gtttcagaca aatgatgaat gtattgaggt gggtggattg 4020

gaaagagaaa aacaccttgt ttaaatgctt tgttgttgtt gttggttttt aaattactta 4080

atcacatatg caggctggat tgatcccaac accaataata ataataataa taataataat 4140

aataataatt tattgttgtt gttattcaat agtacttgct gaagcaggca aggccgactt 4200

tattcagaat tagttattgg tgcagagact tgttgtagag tgttcaatga gagcaagtgg 4260

gtcttaagag ccccagcagg gtgggcgtta gggggatggc aaatgactaa gaggaaagaa 4320

aaatccgggg tagggggact ttgtctaaac agatcagaca agacgcctgt tgagacaggc 4380

caggtgagca gacatcacct caggggctgc acgggatgac tgagggtgat ccagtctagg 4440

agagggagct tgctaaaccg actgaatggg gcttttcacg aaaactaggt tttacaagga 4500

aggcaatgac taggtataga agattctgaa gcct 4534

<210> 2
<211> 4572
<212> DNA
<213> homo sapiens

<400> 2
tcatgaaaaa aatcagggac cccagagttc cttggaagcc aagactgaaa ccagcattat 60

gagtctccgg gtcagaatga aagaagaagg cctgccccag tggggtctgt gaattcccgg 120

gggtgatttc actccccggg gctgtcccag gcttgtccct gctaccccca cccagccttt 180

cctgaggcct caagcctgcc accaagcccc cagctccttc tccccgcagg gacccaaaca 240

caggcctcag gactcaacac agcttttccc tccaaccccg ttttctctcc ctcaaggact 300

cagctttctg aagcccctcc cagttctagt tctatctttt tcctgcatcc tgtctggaag 360

ttagaaggaa acagaccaca gacctggtcc ccaaaagaaa tggaggcaat aggttttgag 420

gggcatgggg acggggttca gcctccaggg tcctacacac aaatcagtca gtggcccaga 480

agacccccct cggaatcgga gcagggagga tggggagtgt gaggggtatc cttgatgctt 540

gtgtgtcccc aactttccaa atccccgccc ccgcgatgga gaagaaaccg agacagaagg 600

tgcagggccc actaccgctt cctccagatg agctcatggg tttctccacc aaggaagttt 660

tccgctggtt gaatgattct ttccccgccc tcctctcgcc ccagggacat ataaaggcag 720

ttgttggcac acccagccag cagacgctcc ctcagcaagg acagcagagg accagctaag 780

agggagagaa gcaactacag accccccctg aaaacaaccc tcagacgcca catcccctga 840

caagctgcca ggcaggttct cttcctctca catactgacc cacggctcca ccctctctcc 900
cctggaaagg acaccatgag cactgaaagc atgatccggg acgtggagct ggccgaggag 960
gcgctcccca agaagacagg ggggccccag ggctccaggc ggtgcttgtt cctcagcctc 1020
ttctccttcc tgatcgtggc aggcgccacc acgctcttct gcctgctgca ctttggagtg 1080
atcggccccc agagggaaga ggtgagtgcc tggccagcct tcatccactc tcccacccaa 1140
ggggaaatgg agacgcaaga gagggagaga gatgggatgg gtgaaagatg tgcgctgata 1200
gggagggatg gagagaaaaa aacgtggaga aagacgggga tgcagaaaga gatgtggcaa 1260
gagatgggga agagagagag agaaagatgg agagacagga tgtctggcac atggaaggtg 1320
ctcactaagt gtgtatggag tgaatgaatg aatgaatgaa tgaacaagca gatatataaa 1380
taagatatgg agacagatgt ggggtgtgag aagagagatg ggggaagaaa caagtgatat 1440
gaataaagat ggtgagacag aaagagcggg aaatatgaca gctaaggaga gagatggggg 1500
agataaggag agaagaagat agggtgtctg gcacacagaa gacactcagg gaaagagctg 1560
ttgaatgcct ggaaggtgaa tacacagatg aatggagaga gaaaaccaga cacctcaggg 1620
ctaagagcgc aggccagaca ggcagccagc tgttcctcct ttaagggtga ctccctcgat 1680
gttaaccatt ctccttctcc ccaacagttc cccagggacc tctctctaat cagccctctg 1740
gcccaggcag tcagtaagtg tctccaaacc tctttcctaa ttctgggttt gggtttgggg 1800
gtagggttag taccggtatg gaagcagtgg gggaaattta aagttttggt cttgggggag 1860
gatggatgga ggtgaaagta gggggtattt ttctaggaag tttaagggtc tcagcttttt 1920
cttttctctc tcctcttcag gatcatcttc tcgaaccccg agtgacaagc ctgtagccca 1980
tgttgtaggt aagagctctg aggatgtgtc ttggaacttg gagggctagg atttggggat 2040
tgaagcccgg ctgatggtag gcagaacttg gagacaatgt gagaaggact cgctgagctc 2100
aagggaaggg tggaggaaca gcacaggcct tagtgggata ctcagaacgt catggccagg 2160
tgggatgtgg gatgacagac agagaggaca ggaaccggat gtggggtggg cagagctcga 2220
gggccaggat gtggagagtg aaccgacatg gccacactga ctctcctctc cctctctccc 2280
tccctccagc aaaccctcaa gctgaggggc agctccagtg gctgaaccgc cgggccaatg 2340
ccctcctggc caatggcgtg gagctgagag ataaccagct ggtggtgcca tcagagggcc 2400
tgtacctcat ctactcccag gtcctcttca agggccaagg ctgcccctcc acccatgtgc 2460
tcctcaccca caccatcagc cgcatcgccg tctcctacca gaccaaggtc aacctcctct 2520
ctgccatcaa gagcccctgc cagagggaga ccccagaggg ggctgaggcc aagccctggt 2580
atgagcccat ctatctggga ggggtcttcc agctggagaa gggtgaccga ctcagcgctg 2640
agatcaatcg gcccgactat ctcgactttg ccgagtctgg gcaggtctac tttgggatca 2700
ttgccctgtg aggaggacga acatccaacc ttcccaaacg cctcccctgc cccaatccct 2760
ttattacccc ctccttcaga caccctcaac ctcttctggc tcaaaaagag aattgggggc 2820
ttagggtcgg aacccaagct tagaacttta agcaacaaga ccaccacttc gaaacctggg 2880

attcaggaat gtgtggcctg cacagtgaag tgctggcaac cactaagaat tcaaactggg 2940
gcctccagaa ctcactgggg cctacagctt tgatccctga catctggaat ctggagacca 3000
gggagccttt ggttctggcc agaatgctgc aggacttgag aagacctcac ctagaaattg 3060
acacaagtgg accttaggcc ttcctctctc cagatgtttc cagacttcct tgagacacgg 3120
agcccagccc tccccatgga gccagctccc tctatttatg tttgcacttg tgattattta 3180
ttatttattt attatttatt tatttacaga tgaatgtatt tatttgggag accggggtat 3240
cctgggggac ccaatgtagg agctgccttg gctcagacat gttttccgtg aaaacggagc 3300
tgaacaatag gctgttccca tgtagccccc tggcctctgt gccttctttt gattatgttt 3360
tttaaaatat ttatctgatt aagttgtcta aacaatgctg atttggtgac caactgtcac 3420
tcattgctga gcctctgctc cccaggggag ttgtgtctgt aatcgcccta ctattcagtg 3480
gcgagaaata aagtttgctt agaaaagaaa catggtctcc ttcttggaat taattctgca 3540
tctgcctctt cttgtgggtg ggaagaagct ccctaagtcc tctctccaca ggctttaaga 3600
tccctcggac ccagtcccat ccttagactc ctagggccct ggagacccta cataaacaaa 3660
gcccaacaga atattcccca tcccccagga aacaagagcc tgaacctaat tacctctccc 3720
tcagggcatg ggaatttcca actctgggaa ttccaatcct tgctgggaaa atcctgcagc 3780
tcaggtgaga tttccggctg ttgcagctgg ccagcagtcc ggagagagct ggagaggagc 3840
cgcattctca ggtacctgaa tcacacagcc aagggacttc cagagattcg ggtgtctagg 3900
cttcaaatca ccctgtccta actctgcaac ctgaaccagc cacttaacct atctatccaa 3960
tggggatagg aatgtccacc acacataggg catgtgagag aaggcctgac ctccatcaga 4020
ggacctcact cagcccttgg cacagtgggc acttagtgaa ttctggcttc cttcaaccag 4080
tttccagctg ttctatcccc ttccattctc tcagtgggtg aaatcgaaga gactgaggac 4140
aataaagaac aaggaaccga actgccggac gtggtggcat gcacctgtaa tcctaccact 4200
ttgcaaggcc aaggtgagag gatcgcttga acccaggagt tccagagcaa cctgggcaac 4260
atagtgagat cctgtctcta ttttttaaaa aagaatgaaa cataggaata agatgtgggt 4320
gaaggactca catgccggct tggtcccact ggtctttgtg gtgaaggagg ggagaggtga 4380
gaggtgggta atccggaaag agaaaagcac cccctccctg gatgaaggct cttctggaga 4440
gagtcaaaga caaataaggg tggggcgcag tggctcatgc ctgttatccc aacactttgg 4500
gaggctgagg tgggaggacc acttgagccc actagttcaa gaccagcctg tgcaacatag 4560
caagaccttg tt 4572

<210> 3
<211> 5136
<212> DNA
<213> mus musculus

<400> 3
actagtgaag gatgaccatg ggtttctgac ggttctgctg ggattacagg cccgcagcac 60

caagcctgct tgatttcgtg ctggggacaa agccagggcc ctgggcatgc aaggccagtg 120
ctctcccatg aaccacagcc ctgagctact tctcatatct ttccaggttg tgactattac 180
ctcatgttac cgctgaattg ttcaagttag tctttgtttc ctcttttttt ttccttgcct 240
caggaagaca aaatttacat ctagtaaaat gtatgaagct ggaattagtt ctgttttgcc 300
tgttacagaa tcaaacctac tagccaagtg ccctgtcagg aaactctact gctatcccaa 360
cactgccttt tcgtctctct gggcctcagc atcttcttag ggctttccca tggagacgaa 420
gtggctagtg taataaagac tgtagttcag tggctagcac attcttggtg cctttggtca 480
gtcagctgta ttctactaat attggtgttg gtaatatttt ccaaacgagt tgtctggtct 540
gggacataga gactgcaagg tcaccaggaa ggggacatgg cctgaaacct ctgtccacct 600
tcctccgacc tgctcttccc taaactccaa tcagctgccc ctcgggtgcc attaattcgg 660
ttcctgatgt tcattgaagt caacaaattt ctcattcatt cattcattca ttcatacatt 720
catttgccct gtccctcatt tataagtagt tgatgcttcc caatggagga ggctcattct 780
agacagactc ccttaaggtg gagtgtgcct ctgtattgct ttatcaggac agagagagag 840
agagagagag agagagagag aggctgagcc cccttcataa agccataacc actgcggacc 900
cacttaattc tgcctttccc atctggtttt agagactgaa acaggaagaa gtcagccagt 960
gtggggagag ggtcgtatca gggacgcaga cacacagccg acccttgttg gcctcccact 1020
ccatctcctc accccccctc ccgtgtgtgt gtgtgtgtgt gtgtgtgtgt gtgtgtgcgc 1080
gcgcgcgtgc gtgcgtgcat gcatgtgtgc aggtgcgtgt gtctttgcag ccctcccttc 1140
agcactgtaa ggtccagaag catgaagaac acacgagata cttggagtcc tacctggcca 1200
tgacaacctt gtttgttgcc tggccttctg caagctccct tccttccctg ggcttcatct 1260
tccctccctg ccaagcccct cttcatcttt accttgaaaa cctctctcta ccccatctcc 1320
ttccccagtt cagagaaccc aggcatccag ccacccaacc ccggccccag cgctgggtaa 1380
acaggaagct gggtgagggg aggaagggtg ttcggaaagt ccccgggcag ggggcaggtg 1440
tgtgggtctg cgggggtggg ggggtctacc cctgaggtat gaaagcccct gccccggtcc 1500
tagttctgag tctggatggg gacacgggga ctgcagggcc tgggtgggag accccagggg 1560
aggggctgcc tcttgctggc tgtggcagga gctacttccc tggtgaccct gttgttggca 1620
gtgcctatca ctgtcctggc tgtgctggcc ttggtgccgc aggatcaggg acgtcgggtg 1680
agtggctgca acgggctcca gagggctgcc tcttgtgact gtttatttac ttatggctgt 1740
gcttctgccc accgcgctca gctggccgct ctccccagag ggaatgtctg gtctgtcttt 1800
gcctctccag gcaatcctag cctgaatttt caagcccctt cctggttggc ttcttttcca 1860
gataacactg cacttgcgtc tctctgcctg catacatcgt ctttgtttgt tcttctagca 1920
agatgcagtc tagggaggac acagcaggcc caggccttgg ggctgggctc tacggtggga 1980
ggggtggagt tgccattagc caaatctgac ctctgggcac tctaacccta cctacccatc 2040
caggttgaga agatcattgg ctcaggagca caggctcaga aaagactgga tgacagcaaa 2100

ccgtcgtgca tcttgccctc accctctagc ctctcagaga ctcctgaccc ccgtctgcat 2160
cctcagagat ccaatgcttc caggaatcta gcctccacat cccagggccc tgttgcgcag 2220
tcctctcggg aggcatctgc atggatgacc atcctgtctc cagctgcgga ttctacacca 2280
gatccagggg ttcaacagct gccaaagggg gaaccagaaa ctgacctcaa ccctgagctc 2340
cctgctgccc acctcatagg taagcatctg gtagaccgaa gagtgctggc tatgtacccc 2400
cacagtaagc gagagtcctt tggctctgct atgacactac tggtactttc ccaactcctc 2460
caccaccaac ttctccctcg gtatgactga ctgctcagga aacaggtaaa aaccggcagg 2520
gatctcgcca ctttagtccc ttcgggtgat agatagcacc gttattcctg cccctccccg 2580
ctaagtacca cagaaggagg aagaccccgc tcctccgctc caggtccctg ctcatcctgc 2640
ccgggtctcc gacctagaga tcacggcccg agcgctcacg cgtgtccctt tctgcaggcg 2700
cttggatgag tgggcaaggg ctcagctggg aggcgagcca agaagaagcg tttctgagga 2760
gcggcgcgca gttctccccc acccacgggc tggcgctgcc acaggacggc gtctattacc 2820
tctactgcca cgtcgggtac aggggcagga cgccccctgc cggccgaagc cgtgctcgct 2880
cgctcacgct gcgcagcgcc ctgtaccgcg cggggggcgc ctacgggcga ggttcccccg 2940
agttgctgct ggagggcgcg gagacagtca cacctgttgt ggaccccatc gggtacgggt 3000
cgttatggta cacgagcgtg gggttcggcg gcctggcgca gctccggagc ggcgagaggg 3060
tctacgttaa catcagtcac cccgacatgg tggactacag gagagggaag accttcttcg 3120
gggcggtgat ggtggggtga cagccatctg tattcattcc tggaggatcg actgacggtg 3180
cgaatgtgtg aatcgtgatg tccgggaccc cgacagtccc gagcggccgg gggcgtggcg 3240
gggggggggg agaacggaat gtaggacacg aattttgaaa ataaagaatg taaactatgc 3300
cggcccttgc cagtgtcttc acggaaatgc agacgtggtt tttgattctg ggacacgtgc 3360
aggtgtggct gacccagctt tgaactgtgg acacctgttg cacccacgtc ctggctctaa 3420
ggccagggct ccaagagggc ggaagaaggg acaattaaac cctgagactt ctgccagcac 3480
cctgattgcc aggatgctga aaggttaggg aggttggttg aggtttgtta gcagctgcgg 3540
gtcggtcaaa gaagaaacga aaggaaagat attagactca cggattttcc taatccttat 3600
ttctatccta tctagtgtta gggggtgaaa ccagggcgag aaagggtggg agaaagaatc 3660
cacaaagtag caaagacctg gtccccacaa gaccccctca catacaccct tgctcctggc 3720
ttctgcctct gtgaggagga gccaacagga gaaggtaggc aggattcgtt cccagagaga 3780
aaaactacaa agactgtgga acaaagctcc gtgggttggg cccatcagtt cagttgggcc 3840
tgataggtta tatgtatgta gtatgtatgt aatgtatgta tggatggatg tatgctctat 3900
ctatctatct atctatctat ctatctatct atctatctat ctatctatct atctatctat 3960
ctatctatag tccgggtctc atatatagtc ctgggtcacc tggaactctc caggctgggg 4020
gctaaaggca tggaccacca cgggttctga cagcccttct ttaaggggca gcaggtcagt 4080
cgcaggcacg ttaaggggca gactaatgaa gaaagcaaag gagctgaaaa tggcaggggt 4140

ggggtggggg aggggattgt gtccgaggag gaggagttcc aggaggaagc cgatgccctg 4200
gggtcctttc tagaaatgga atgtcttcac ctttggttca cacaaagttg ggcaagtggg 4260
aggcccctac gatgagtaac agcagccccc ccccccccac gccatggctg gaggaggcag 4320
agggggaccc aggcactatc taggtcaccc cagtgggtca cccctacccc ccaccccacc 4380
ccagcatacc acgtggttgg tttttccctc tctacacggg tgcaggaagg cccaaccaac 4440
aaagagccca ctggggtttc attttctttt ctcttctctt ctcttctctt ctcttctctt 4500
ctcttctctt ctcttctctt ctcttctctt ctcttctctt ctctttttta aaagatttat 4560
ttatttatta tatgcaagta cactgtagct gtcttcagac actccagaag agggagtcag 4620
aagacttgtt acggatagtt gtgagccaca atgtggttcc tgggatttga actcaggacc 4680
tttggaagag cagttggtgc tcttaaccac tgagccatct ctccagcctc ttctcttctc 4740
ttctcttctc ttctcttctc ttctcttctc ttctcttctc ttctcttctc ttctcttctc 4800
ttctctcctc tcctctcctc tcctctcctc tcctctcctc tcctttccct tcccttcctt 4860
tcctcttctt ttctctgttc tttcttcctt ctttcccttt ttctcctcct cctccccctc 4920
ctccccctcc tcttcctcct cttcttcctt ctcttcctct ctctcctctt ccttctccca 4980
ctgaccttga agccctcaac aatttacctg cccctacctc cgaagtgttg ggattaaaag 5040
catggctaca cacccactcc ttatgtgctt tggaccttct gcacagagcc ttccagtggg 5100
gtgagagggt gaccaatttt ggctaaagtc tagtca 5136

<210> 4
<211> 1946
<212> DNA
<213> artificial sequence

<220>
<223> neo cassette

<400> 4
taccgggtag gggaggcgct tttcccaagg cagtctggag catgcgcttt agcagccccg 60
ctgggcactt ggcgctacac aagtggcctc tggcctcgca cacattccac atccaccggt 120
aggcgccaac cggctccgtt ctttggtggc cccttcgcgc caccttctac tcctccccta 180
gtcaggaagt tcccccccgc cccgcagctc gcgtcgtgca ggacgtgaca aatggaagta 240
gcacgtctca ctagtctcgt gcagatggac agcaccgctg agcaatggaa gcgggtaggc 300
ctttggggca gcggccaata gcagctttgc tccttcgctt tctgggctca gaggctggga 360
aggggtgggt ccgggggcgg gctcaggggc gggctcaggg gcggggcggg cgcccgaagg 420
tcctccggag gcccggcatt ctgcacgctt caaaagcgca cgtctgccgc gctgttctcc 480
tcttcctcat ctccgggcct ttcgacctgc agccaatatg ggatcggcca ttgaacaaga 540
tggattgcac gcaggttctc cggccgcttg ggtggagagg ctattcggct atgactgggc 600
acaacagaca atcggctgct ctgatgccgc cgtgttccgg ctgtcagcgc aggggcgccc 660
ggttcttttt gtcaagaccg acctgtccgg tgccctgaat gaactgcagg acgaggcagc 720

gcggctatcg tggctggcca cgacgggcgt tccttgcgca gctgtgctcg acgttgtcac 780
tgaagcggga agggactggc tgctattggg cgaagtgccg gggcaggatc tcctgtcatc 840
tcaccttgct cctgccgaga aagtatccat catggctgat gcaatgcggc ggctgcatac 900
gcttgatccg gctacctgcc cattcgacca ccaagcgaaa catcgcatcg agcgagcacg 960
tactcggatg gaagccggtc ttgtcgatca ggatgatctg gacgaagagc atcaggggct 1020
cgcgccagcc gaactgttcg ccaggctcaa ggcgcgcatg cccgacggcg aggatctcgt 1080
cgtgacccat ggcgatgcct gcttgccgaa tatcatggtg gaaaatggcc gcttttctgg 1140
attcatcgac tgtggccggc tgggtgtggc ggaccgctat caggacatag cgttggctac 1200
ccgtgatatt gctgaagagc ttggcggcga atgggctgac cgcttcctcg tgctttacgg 1260
tatcgccgct cccgattcgc agcgcatcgc cttctatcgc cttcttgacg agttcttctg 1320
agcgggactc tggggttcga aatgaccgac caagcgacgc ccaacctgcc atcacgagat 1380
ttcgattcca ccgccgcctt ctatgaaagg ttgggcttcg gaatcgtttt ccgggacgcc 1440
ggctggatga tcctccagcg cggggatctc atgctggagt tcttcgccca ccccgaaatt 1500
gatgatctat taaacaataa agatgtccac taaaatggaa gtttttcctg tcatactttg 1560
ttaagaaggg tgagaacaga gtacctacat tttgaatgga aggattggag ctacgggggt 1620
gggggtgggg tgggattaga taaatgcctg ctctttactg aaggctcttt actattgctt 1680
tatgataatg tttcatagtt ggatatcata atttaaacaa gcaaaaccaa attaagggcc 1740
agctcattcc tcccactcat gatctataga tctatagatc tctcgtggga tcattgtttt 1800
tctcttgatt cccactttgt ggttctaagt actgtggttt ccaaatgtgt cagtttcata 1860
gcctgaagaa cgagatcagc agcctctgtt ccacatacac ttcattctca gtattgtttt 1920
gccaagttct aattccatca gaagct 1946

<210> 5
<211> 5531
<212> DNA
<213> artificial sequence

<220>
<223> right arm

<400> 5
gctcaggctg ctgctttggg tccctgctcc caagtgagtt ttccacggag cctctgccat 60
atcttgactg cggtacatca actcagacat ttaggtccca cagccctgct tccaggattt 120
ctcccaatcc gtatgactcc ccggtcttcc aaggattccc ctcccccacc ctcccactcc 180
taaacactct cccaccctcc agtggagtca cttctcccca gaaccctcat ctctctccac 240
ccttggatgg atctccctag ctcatccttt gggtctctcg gcagttaagc tgcctcactc 300
ccgtgaatcc accatgtctc tgggagctgc ctgctcctca tgtctctttg ctctgcccgg 360
atcccatgga ccaactgagg cctctgtccc ctgctccact ccttaaagag accaggaagt 420
ttctccccca acgcaagaca gacacaagca gacagaatct cagagaaaag aggtttattg 480

ggtttcacag atggtgcagg gggtccctgt ggatgtctag ccaagcagtg gctggctttt 540
agagcttcgt gctttcttct agaacccctt ggtcacccac atctaattct ctcgccatct 600
ctctcttaga tgggtcctgt ctgaggtgag acacctttgt gtctgggacc tagttgtcat 660
ctgacggctt tctatttttc ccctctttct attctctata aataaataac ttaacttttc 720
tctccataaa tagtcaacct tcccctgttc ccagccacct ctcccatgtc tgtccctcct 780
tcatgtccag gtctctgtcc gacctagacc cacaaaaacc ctgctctgtg caggaacttc 840
taggtaggct tgagtcgtcc cctgagcatc tggagtggcc ctggggaaga tgtagtcccc 900
tgaagtgtct cagactcccg tgagagctcc aggttatttt agtggggaag gctggagact 960
gtggggatgt gacccttgaa acaacggtca ggatggaggc ctggaatcca attcttgggt 1020
ttctttagaa tctacagtgc aaaggctcca aagaatacac tgctggggct gaagtgtaga 1080
tgggagatgc cgtcggtgtg ggtggacagc tggtctccct tactgagcag gaacacagcc 1140
ccctggtaca ttgagcgcac ccacggtcct tgaagtcccg gatacacaga cttctgcgca 1200
ctgaggagag gcacatggaa ggggtattgg gaggaaaaga gctggacctc gtgtgccagg 1260
tagatgggag tgggaatggc cctgggggag cagctttctc cagagaaaac cacctgggag 1320
tagacaaagt agaggccact ggtggggatc aggagggagt tgttgctcaa agagaagcca 1380
tgtcggagaa aggcacgatc cgtgcttgct ctccagagca gtgagttctg cttgctgggg 1440
taccctggga agaggcacaa gacattgggg ggctatcaag atcagaggtc cccatccctg 1500
aggagcaggc actggacaag tgggatggtt ggtagggaga tgggagtggg gtgcctgccc 1560
ttaggggtaa gaaaagttgg tgtggggaga tgggaagatc gcctgagccc ctgggcaagt 1620
aggacaaaag gcagggactg gacctctcct ctggaggcag aagtttacca acaaggtgag 1680
cagcaggttt caggatgcca tgggtcaagt gcttctgagg gagtggatgg gctgtcctgg 1740
cagcggagaa gcggacacca gagagtccct gggacagaag agagtggaga ggactctagg 1800
actctgagtt agctaggcca ccccagcacc cccaatctct tgctgcctca cctgggcccc 1860
tagaggcagg gccagcagca gccccaggag gaagacagga ggggtgccaa gcaccctcaa 1920
gaggtggaga cggccgagca gtgtcatgtg gagaacctgc tgagagagag agagagagtg 1980
tgtgtgtgta gcggaggcgg ggcacacagc ggaagacaga ccttacctcc cagctgagac 2040
agccaccctg agagacaggg cgacagacag aaaaggggac aggcagggga accctgaagt 2100
gagcagggat aaggagagac agaggggaag aggaaagctc caccatagaa tcagacaatg 2160
gctaacagag gcagagagag agaagagaga gagagagaga gagagagagg gagagagaga 2220
gagagagaga gagagagaga gagagagaga gagagagaga gagagactga taaggccgcc 2280
acaggaacag accaaaaatc aaagccacga caggaggacc agacccatta aggctgggga 2340
tccaggcagg ttgtatagaa aaggctgtgg ctcaagaaag gaggtaggat cctgagggat 2400
gcctgatacc agggtaagcc caaggagaca gggtaggaga ggctcacctg ctgtgcgggg 2460
tcctgggcgc tggcgctcgg gtccctttat agaggaagcg gcagtgtggc aggcagcggg 2520

cgggttctag gtcggggctg gggcttgggg aagcccccag ggcttagaag atgctgctgt 2580
ttcagtcgaa ggcaggaaag gctggggcct aggagagaac cgcaggctaa gggcttagac 2640
tactgcgttc tgggaaaggg agtcgggtcc ggggaactgt gggccgcagg gactagcagg 2700
gagctgggtc aggcccagga gttccagtga acggccgagc aattcgtgga gagggtgcag 2760
ctgggctgtg actggaagcc tggttccctg aagagtgatg gtttatatca cttctgcacc 2820
cttgactgct cacaggcttc tctgcacatt tccctctctg tcgatcatgg tcatgtctgg 2880
agggctccga agccacatgt ggttagcggc acctttgttt tttcaggttc gaacccaggg 2940
ctctggatgc ttgacatgcc tgccgcccct tcaggcctat ttcctctctt gtctttgggg 3000
gttcaagtca cagttattcc aatatatctg tattttctgg tcatctgcca gttttttttt 3060
tttttttttt ttttttttgc ttttttcgag acagggtttc tctgtgtagc cctggctgtc 3120
ctgaaactca ctctgtagac caggctggcc tagaactcag aaatccgcct gcctctgcct 3180
cccaagtgct gggattaaag gtgtgtgcca ccactgccca gctttttttt ttttttctgc 3240
cagtttcatc tgccagtttc atgtcaagaa gttccattac agtgccctct ccccaaatct 3300
ggggcatcct ggtgtgtaac aatcttaact gtgtcccctt actctctgga gcggcagttc 3360
tcaacctgtg ggcaaggtcc ctttggcaaa cctctattta aaacaaatat ttacattatg 3420
attcataata gcagcgaaat tacagttatg aaatagcaac gaaaataatt gtatggttgg 3480
ggtcaccaca acatgaggaa ctgtattaaa gggtcatgga gttaggaagg ttgagaaccc 3540
tgtcctagaa tgttccaggt ctggaagatg atttgggggt attctcttag aaccccaaga 3600
tcatgtaacc ccaaatgtac accccaggct tccaatgctc tgtacagagg gagcttcaga 3660
attctacaca ggtgaaggca gttttttgtt tgtttgtttg tttgtttttc gagacagggt 3720
ttctctgtat agccctggct gtcctggaac tcactttgta gaccaggctg gccccaaact 3780
cagaaatccg tctgcctctg cctcccaagt gctgggatta aaggtgtgtg ctaccactgc 3840
ccaacaaagg acatctgagc agtgcttctc tattggaagg cctgggacac agctcaggta 3900
gctgcttgca ggcatgtgtg agcccctgag tccatcccca cattcccagc agtcacattg 3960
atgatttctg tcttaagaca aaattttaaa atattgtttt gaggattgga gaaatggctt 4020
agggttctga gcactagatg ttcttccaga ggaccagtgt taattcccag catctgcatg 4080
ttggcttatt acatctgtcc ttgcagtttg tggggatgtg acatcctctt ctggcctttg 4140
caggcactat actcaaatgg tgctgagaca tacatgcagg caaaaatcct atacacacag 4200
cagaacattt aaaaacatat ctatttatgt gagtacatac aagtgccaca ggccacatgt 4260
ggaggtcaga gtacagctct ttttttcttt aatatataat tcatttttat ttcatgtaaa 4320
ttggtgtttt gcctgcttgt atgcctgtat gaggatgtca gctctcctgg aacaggagtt 4380
atagacagtt gtaagctgct gtatgggttc tgggaactga actcaggcca ttaggtaaag 4440
ccagtaagca cctttgccca ctgagccatc ttgctggccc aacttccttc tctttctttc 4500
tttctttctt tctttctttc tttctttctt tctttctttc tttccttcct tccttccttc 4560

cttccttcct tccttccttc cttccccccc ccctctcttt ctttttgttt ttcaagacag 4620
ggtttctctg tgtagccctg gttgtcctgg aactcacttt ttagaccagg ctggccttga 4680
aactcagaaa tccgcctgcc tctgcctccc aagtgctggg attaaaggca tacaccacca 4740
ttgcctggct ctttttcttc ttattattat tattatcaaa gtgtttgatt ttttttcaaa 4800
atttactctt taaagttatt ttattattat attttatata ttacttatac acacatatat 4860
acatatacat gtatacatac atatacatag agacaggctc tcactatgta gccctagctg 4920
ccctggaact caatatgtgc atcaggatgg ccttgaactc acagaaatca cctacttcaa 4980
ccttccaagt gctgggatta atggtgtgtg caccgaatgt catccatcta tcaagctttc 5040
ttatacaacc caggatcccc tgtgcagagg acggtgccac tcatggtggt cttgggcctc 5100
ctacatcaat taacaccccc cctccccgcc tcccaaacac atacagcctg ggcaatccct 5160
cagctgagac tccttttcag gtgactctag actgtcttaa gtggatagcc aaagtcacaa 5220
ggatactcct gatggctgtt aggttggtct agtttctgtc tgagaaacac tgagtaggaa 5280
actcctgttc ggaaccagtt ttctctcagc ttgtaagtta cagctctcaa aaagcaaagt 5340
ttttgaagat gatttattat ttattttttg ctcgtttttt tttttaagat ggagatagat 5400
gacaacagct ggccacctca gatgtcaccg ctgttgccac atggcatcag tcagactatt 5460
gtagcaggct gcagctggtg atggtggcag tcaggctggc cactgggcac actgctgttc 5520
tcaggcccaa a 5531

<210> 6
<211> 1848
<212> DNA
<213> artificial sequence

<220>
<223> HS-TK cassette

<400> 6
cagtgtggtt ttcaagagga agcaaaaagc ctctccaccc aggcctggaa tgtttccacc 60
caatgtcgag cagtgtggtt ttgcaagagg aagcaaaaag cctctccacc caggcctgga 120
atgtttccac ccaatgtcga gcaaaccccg cccagcgtct tttgtcattg gcgaattcga 180
acacgcagat gcagtcgggg cggcgcggtc cgaggtccac ttcgcatatt aaggtgacgc 240
gtgtggcctc gaaacaccga gcgaccctgc agcgacccgc ttaacagcgt caacagcgtg 300
ccgcagatct tggtggcgtg aaactcccgc acctcttcgg ccagcgcctt gtagaagcgc 360
gtatggcttc gtaccccggc catcaacacg cgtctgcgtt cgaccaggct gcgcgttctc 420
gcggccatag caaccgacgt acggcgttgc gccctcgccg gcagcaagaa gccacggaag 480
tccgcccgga gcagaaaatg cccacgctac tgcgggttta tatagacggt ccccacggga 540
tggggaaaac caccaccacg caactgctgg tggccctggg ttcgcgcgac gatatcgtct 600
acgtacccga gccgatgact tactggcggg tgctgggggc ttccgagaca atcgcgaaca 660
tctacaccac acaacaccgc ctcgaccagg gtgagatatc ggccggggac gcggcggtgg 720

taatgacaag cgcccagata acaatgggca tgccttatgc cgtgaccgac gccgttctgg 780
ctcctcatat cgggggggag gctgggagct cacatgcccc gcccccggcc ctcaccctca 840
tcttcgaccg ccatcccatc gccgccctcc tgtgctaccc ggccgcgcgg taccttatgg 900
gcagcatgac cccccaggcc gtgctggcgt tcgtggccct catcccgccg accttgcccg 960
gcaccaacat cgtgcttggg gcccttccgg aggacagaca catcgaccgc ctggccaaac 1020
gccagcgccc cggcgagcgg ctggacctgg ctatgctggc tgcgattcgc cgcgtttacg 1080
ggctacttgc caatacggtg cggtatctgc agtgcggcgg gtcgtggcgg gaggactggg 1140
gacagctttc ggggacggcc gtgccgcccc agggtgccga gccccagagc aacgcgggcc 1200
cacgacccca tatcggggac acgttattta ccctgtttcg ggcccccgag ttgctggccc 1260
ccaacggcga cctgtataac gtgtttgcct gggccttgga cgtcttggcc aaacgcctcc 1320
gttccatgca cgtctttatc ctggattacg accaatcgcc cgccggctgc cgggacgccc 1380
tgctgcaact tacctccggg atggtccaga cccacgtcac cacccccggc tccataccga 1440
cgatatgcga cctggcgcgc acgtttgccc gggagatggg ggaggctaac tgaaacacgg 1500
aaggagacaa taccggaagg aacccgcgct atgacggcaa taaaaagaca gaataaaacg 1560
cacgggtgtt gggtcgtttg ttcataaacg cggggttcgg tcccagggct ggcactctgt 1620
cgatacccca ccgagacccc attggggcca atacgcccgc gtttcttcct tttccccacc 1680
ccacccccca agttcgggtg aaggcccagg gctcgcagcc aacgtcgggg cggcaggccc 1740
tgccatagcc actggccccg tgggttaggg acggggtccc ccatggggaa tggtttatgg 1800
ttcgtggggg ttattatttt gggcgttgcg tggggtcagg tccacgac 1848

<210> 7
<211> 33
<212> DNA
<213> artificial sequence

<220>
<223> lox P site

<400> 7
ataacttcgt ataatgtatg ctatacgaag tta 33

<210> 8
<211> 335
<212> DNA
<213> artificial sequence

<220>
<223> human/mouse right junction fragment

<400> 8
ttgagtcctg aggcctgtgt ttgggtccct gcggggagaa ggagctgggg gcttggtggc 60
aggcttgagg cctcaggaaa ggctgggtgg gggtagcagg gacaagcctg ggacagcccc 120
ggggagtgaa atcacccccg ggaattcaca gaccccactg gggcaggcct tcttctttca 180
ttctgacccg gagactcata atgctggttt cagtcttggc ttccaaggaa ctctggggtc 240

cctgattttt ttcatgagct caggctgctg ctttgggtcc ctgctcccaa gtgagttttc 300

cacggagcct ctgccatatc ttgactgcgg tacat 335

<210> 9
<211> 21
<212> DNA
<213> artificial sequence

<220>
<223> primer

<400> 9
atgtaccgca gtcaagatat g 21

<210> 10
<211> 21
<212> DNA
<213> artificial sequence

<220>
<223> primer

<400> 10
ttgagtcctg aggcctgtgt t 21

<210> 11
<211> 21
<212> DNA
<213> artificial sequence

<220>
<223> primer

<400> 11
tgttgtatag gaccctgaga a 21

<210> 12
<211> 1227
<212> DNA
<213> artificial sequence

<220>
<223> DTalpha cassette

<400> 12
   atcgataagc ttgatatcga attcctgcag cccgaccgaa caactccgcc gcgcgcgctc 60
   cgtgtaggcc ggtgcgggcg gccccgtagc gcaagggagg gcgggaaagg aaggggcggg 120
   acacaagggc gaatctataa agggcgtcac tcagccagtt ctctcctcag aagcgccgag 180
   agcgcgaccg ggacggttgg agaagaaggt ggctcccgga agggggagag acaaactgcc 240
   gtaacctctg ccgttcagga tctgcgacct gcaggtcctc gccatggacc ctgatgatgt 300
   tgttgattct tctaaatctt ttgtgatgga aaacttttct tcgtaccacg ggactaaacc 360
   tggttatgta gattccattc aaaaaggtat acaaaagcca aaatctggta cacaaggaaa 420
   ttatgacgat gattggaaag ggttttatag taccgacaat aaatacgacg ctgcgggata 480
   ctctgtagat aatgaaaacc cgctctctgg aaaagctgga ggcgtggtca aagtgacgta 540
   tccaggactg acgaaggttc tcgcactaaa agtggataat gccgaaacta ttaagaaaga 600
   gttaggttta agtctcactg aaccgttgat ggagcaagtc ggaacggaag agtttatcaa 660
   aaggttcggt gatggtgctt cgcgtgtagt gctcagcctt cccttcgctg aggggagttc 720
   tagcgttgaa tatattaata actgggaaca ggcgaaagcg ttaagcgtag aacttgagat 780
   taattttgaa acccgtggaa aacgtggcca agatgcgatg tatgagtata tggctcaagc 840
   ctgtgcagga aatcgtgtca ggcgatctct ttgtgaagga accttacttc tgtggtgtga 900
   cataattgga caaactacct acagagattt aaagctctaa ggtaaatata aaatttttaa 960
   gtgtataatg tgttaaacta ctgattctaa ttgtttgtgt attttagatt ccaacctatg 1020
   gaactgatga atgggagcag tggtggaatg cagatccact aggatctaac ttgtttattg 1080
   cagcttataa tggttacaaa taaagcaata gcatcacaaa tttcacaaat aaagcatttt 1140
   tttcactgca ttctagttgt ggtttgtcca aactcatcaa tgtatcttat catgtctgga 1200
   tcgtagttct aggggggatc cactagt 1227

<210> 13
<211> 705
<212> DNA
<213> artificial sequence

<220>
<223> probe

<400> 13
gaattcgatt aggaagaata gccattggga gatggtgtta catctttcct ttgagattcc 60
tagatatcat tagggtggtt actccctcag ccccagcctc ccggttcacc ctgtactggt 120
aaaggaatgt ccacctcctc caggggcccc ctctgcggtg ccgccttaag gttcatcatc 180
ttgtggtttg tgcataggca tgggctccca gcacctcgag tgccagataa ctctggggtg 240
tctcagggca cctctcttgg tgtctggcag ctatatgcag caggatatcg atgtgctacc 300
aaaggagtag ggaagtcaga ggtagtcaga ctaaatctgc taacagatgc tgacaggaag 360
tgggtgagag aagctgatgt tagcaaagcc ttggggcatc aaggagaact tcccacagaa 420
ggcagcattc tggcattggc aggagggcag gagggactga ggtgaaggtc tctcggtgcc 480
actgtgacca agagagaaag gggtgtagaa gactacaccc caatctttga acagagagga 540
agctggtagc taggatggag acagaggtgg gtgagtctgg tgcctgggtc cctatgcatg 600
ttttgttaac caaattcagg gactttgtgg tactcccccc ccagtgtttc ccccagaaca 660
aatggggatc acaatctaag tgggtttcta atcactagtg aattc 705

<210> 14
<211> 335
<212> DNA
<213> artificial sequence

<220>
<223> probe

<400> 14

ttgagtcctg aggcctgtgt ttgggtccct gcggggagaa ggagctgggg gcttggtggc 60
aggcttgagg cctcaggaaa ggctgggtgg gggtagcagg gacaagcctg ggacagcccc 120
ggggagtgaa atcacccccg ggaattcaca gaccccactg gggcaggcct tcttctttca 180
ttctgacccg gagactcata atgctggttt cagtcttggc ttccaaggaa ctctggggtc 240
cctgattttt ttcatgagct caggctgctg ctttgggtcc ctgctcccaa gtgagttttc 300
cacggagcct ctgccatatc ttgactgcgg tacat 335

## Description

The present invention relates to a transgenic mouse, in particular a knock-in mouse, tissues, cells and/or cell lines derived from said transgenic mouse and a recombinant vector intended for the generation of such a mouse wherein the endogenous murine TNF-α gene is replaced by the human homologue. Furthermore, the invention relates to stem cells, preferably murine embryonic stem cells comprising said recombinant vector as well as to a method for screening, investigating and/or preclinical testing of compounds and/or drug candidates for human anti-TNF-α therapy using said transgenic mouse and/or tissues, cells and/or cell lines derived thereof, as well as compounds discovered or developed using this method. TNFα exists as a soluble homotrimer of 17kD protein subunits (Smith R.A. et al. 1987, J. Biol. Chem. 262: 6951-6954) and as a membrane bound 26kD precursor form (Kriegler M. et al. 1988, Cell 53: 45-53). For review of TNFα see for example Old L.J. 1985, Science 230: 630-632; Wajant H. et al. 2003, Cell Death Differ. 10: 45-65. The major source of TNFα are the cells of the monocyte/ macrophage lineage, with T lymphocytes, B lymphocytes, neutrophils, mast cells and endothelium all contributing under different circumstances. All potential noxious stimuli, ranging from the physical (UV-light, X-radiation, heat) to the chemical and immunological can rapidly induce TNFα production and release (Vassalli P. 1992, Annu. Rev. Immunol. 10: 411; Aggrarwal B.B. et al. 2000, In The Cytokine Reference ed. OJ, FM. New York: Academic Press). In vivo TNFα is the most rapidly produced pro-inflammatory cytokine, with serum levels detectable in mice in 30 min (Tracey K.J. et al. 1987, Nature 330: 662-4). TNFα is thought to coordinate the cytokine response to injury and to various infections activating receptors of innate immunity, and it acts as a fire alarm system in vivo.

By promoting the inflammatory response TNFα can be seen as the master regulator of the activities of other cytokines. Thus, TNFα plays also a key role in autoimmune disorders such as rheumatoid arthritis (RA), ankylosing spondilitis (AS), inflammatory bowel disease (IBD), acute and chronic immune diseases associated with transplantation and others. Hence, the anti-TNF-α therapy has emerged as an efficient treatment of these autoimmune diseases in humans (Feldmann M. et al. 2001, Annu. Rev. Immunol. 19: 163-196).

At present the only drugs that are in clinical practice or in clinical trials to block TNFα are biologicals, protein-based drugs, either based on antibodies against TNFα or based on TNFα receptor. For example US patent 6,015,557 discloses the use of a chimeric monoclonal antibody binding to TNFα, called Infliximab (trade name: Remicade).

US Patent 6,258,562 shows the sequence of a fully human monoclonal antibody binding to TNFα, called Adalimumab (trade name: Humira). A drug based on the TNFα-receptor is for instance Etanercept (trade name Enbrel) which is a fusion protein comprising the TNFα-receptor and the F_{c} component of human immunoglobulin G1 (IgG1) also disclosed in US patent 6,015,557.

These agents have the major advantage of specificity but also significant disadvantages, including the need for repeated injection and their relative high costs compared to small organic chemical drugs. Thus, the development of novel and better anti-TNFα drugs is highly desirable.

One of the problems of anti-TNFα drug development is the lack of an appropriate animal model for screening, investigating and preclinical testing. At present most of the clinically used anti-TNFα drugs are not active in mice because they recognize human and not murine TNFα. Only Etanercept is able to block murine TNFα as well, so that it can be evaluated in vivo, by blocking acute liver toxicity induced by LPS-Dgal. However, Infliximab, Adalimumab and some newly developed drugs cannot be compared with Etanercept in vivo. But these experiments are needed to understand the differences in efficacy and outcome of the different anti-TNFα therapies, for instance why Infliximab is effective in Crohn's disease, and Etanercept is not.

Usually the anti-TNFα drugs are only tested by neutralization of TNFα activity in vitro by blocking TNFα-mediated killing of two cell lines L929 and WEHI-231 which are sensitive to TNFα. But in vitro testing cannot predict the reaction of a complex biological system.

Thus, it is the object of the present invention to provide an appropriate animal model for screening, investigating and preclinical in vivo testing of anti-TNFα drugs and drug candidates. It is another object of the invention to provide a method for screening, investigating and preclinical in vivo testing of anti-TNFα drugs using the appropriate animal model.

It is a further object of the invention to provide cells, cell lines and tissues derived from such an appropriate animal model for a better preclinical in vitro testing of anti-TNFα drugs and drug candidates and to provide a method for screening, investigating and preclinical in vitro testing of anti-TNFα drugs and drug candidates using the derived cells, cell lines and tissues of said appropriate animal model.

This problem is solved by the subject matter as characterized in the independent claims and in the description. Further and preferred embodiments of the invention are to be found in the dependent claims and in the description. In accordance with the invention, the solution to the above-mentioned problem is provided by generation of a transgenic mouse, preferably a knock-in mouse, wherein the complete endogenous murine TNFα-gene including its upstream regulatory sequences represented by SEQ ID No: 1 is replaced by the human homologous sequence represented by SEQ ID No: 2 or a mutant thereof, wherein the mutant is characterized by deletion, addition, substitution, and/or inversion of one or more nucleotides so that the functional properties of the modified human TNFα-gene are conserved.

Thus the invention relates to transgenic mice wherein the human TNFα gene is introduced in the genome instead of the murine gene. The sequence of the human TNFα gene is shown in SEQ ID No: 2. The invention further relates to any mutated sequence, wherein the DNA can be translated into a fully functional human TNFα protein. Mutation are for example every single base exchange which does not change the amino acid sequence due to degeneration of the genetic code, but also mutation which results in changes of the amino acid sequence, like deletion, addition or substitution are included, or any mutation in non-coding regions of the gene, which may change its regulation, provided that the TNFα protein retains its biochemical activity.

The mice according to the invention are incapable of expressing endogenous TNFα due to full replacement of the gene by its human homologue. The transgenic mice of the present invention are human TNFα-Knock-in mice. Such true knock-in mouse has many advantages for instance compared to a mouse where the human TNFα gene is put in an arbitrary place somewhere else in the genome and which would be easier to produce. The latter mouse may show obvious or hidden gene defects due to arbitrary integration of the human gene into the mouse genome with potential disruption of another gene or function. Further the endogenous TNFα gene had to be knocked out as well, if drugs to human TNFα induced diseases shall be tested effectively.

The transgenic mutation is present in somatic and germ cells of the TNFα-Knock-in mouse according to the invention. Transgenic mice at every stage of development, including embryonic, juvenile, adolescent and adult and their offspring are included in this invention. The transgenic mice according to the invention are further viable, fertile and capable of transmitting the transgenic modification, in particular the human TNFα-Knock-in, to its offspring. In a preferred embodiment of the invention the transgenic mice are homozygous for the transgenic mutation. It is an advantage of the present invention to breed the transgenic mice as a homozygous line, where all mice show the desired genotype.

In a preferred embodiment the transgenic mouse is an established inbred strain of lab mice. Preferably the mouse is a C57BI6 mouse which is preferably used for experimental immunology in mice. These mice are very robust and easy to breed.

The transgenic mice according to the invention are grossly normal and possessed apparently normal immune system and normal protective functions. As the murine TNFα gene is completely knocked out in the homozygous transgenic mice the physiological and the pathogenic TNF-α functions are mediated completely by the genetically introduced human TNF-α. Thus, a "humanized" TNFα mouse can be engineered by the invention which can be used as in-vivo animal model to compare the efficacy of human anti-TNFα drugs and methods. Acute toxicity mediated by human TNFα in these mice, for instance using the LPS-Dgal model, can be blocked by antibodies and drugs specific for human TNFα, but not by substances specific for murine TNFα.

It is a further object of the invention to provide cells, tissues and/or cell lines which are derived from the human TNFα-Knock-in mouse according to the invention. In particular human TNFα knock-in mice are sacrified and cells and/or tissues are extracted according to standard protocols. Cells and/or tissues can be derived from somatic and/or from germ cells. Preferred examples for tissues and/or cells which are derived from human TNFα-Knock-in mouse according to the invention are lymphoid organs, tails, blood , mouse embryonic fibroblasts (MEF), bone marrow derived macrophages or dendritic cells, total splenocytes or other sorted cell populations. Cells and tissues are cultivated under appropriate culture conditions. Culture conditions are based on the tissue cells are derived from and the intended use. Several standard protocols are known in the art (Current Protocols in Immunology; Copyright 2007 by John Wiley and Sons, Inc.).

Cell lines can be derived from the cultivated cells and/or tissues by transforming them to unlimited growing. Methods and protocols for cell transformation are known by the skilled person. In particular bone marrow derived macrophages can be transformed using the protocol of Cox GW et al., J Natl Cancer Inst. 1989, 81:1492-1496. Thus, stable cell lines with macrophage/monocyte properties are obtained which is particularly useful for studying TNFα.

It is another object of the invention to prepare the human TNFα-Knock-in mouse according to the invention. Thus, a method of production of the transgenic mouse is included comprising the steps of
a) generating an appropriate recombinant vector comprising the human TNFα-gene
b) transfecting said recombinant vector of step a) into murine embryonic stem cells;
c) selecting an ES clone with desired mutation in one allele
d) microinjecting an embryonic stem cell of step c) into a murine blastocyst and transferring said blastocyst into a receptor mouse;
e) selecting the offspring until a human TNFα Knock-In mouse is obtained.

The TNFα-Knock-in mouse according to the invention can be created by methods of gene targeting based on homologous recombination in embryonic stem cells. In a preferred embodiment the recombinant vector is a vector for homologous recombination comprising at least, the target gene, marker genes for positive and/or negative selection and the sequences needed for homologous recombination. The recombinant vector can be transfected into murine ES cells using the standard protocols. After transfection the ES cell are cultured under selection culture conditions to amplify only the ES cell clones which has undergone correct homologous recombination. Amplified cells can be further characterized using molecular biology, such as genomic Southern blot analysis, polymerase chain reaction (PCR) and/or DNA sequencing. ES cells with the desired genetic modification are microinjected into murine blastocysts. Living embryos are transferred into a female pseudo pregnant recipient mouse. The offspring of the female recipient mouse is further analysed and mice which are positive for the transgenic mutation are intercrossed until the transgenic mutation is located in the germ line and homozygous human TNFα Knock-In mice are obtained.

It is another object of the present invention to provide a recombinant vector for homologous recombination in murine ES cells, wherein the target sequence is the human TNFα-gene including its upstream regulatory sequences represented by SEQ ID No: 2. The genetic construct is based on the genomic clone of human TNFα which is shown by Nedospasov S.A. et al 1986; Nucleic Acid Res. 14(19):7713-7725.

In addition to the human TNFα sequence represented by SEQ ID No: 2 any mutated human TNFα sequence including alterations in non-coding and regulatory elements can be used which can be translated into a fully functional human TNFα protein which retains its biochemical activity. Mutation can be introduced by the methods of molecular biology known to the skilled person or can appear according to natural mechanisms, for instance mistakes in DNA-replication and/or DNA-repair.

The recombinant vector according to the invention comprises the following components in functional combination: the target gene comprising the genomic and/or cDNA sequence encoding the human TNFα-gene including its upstream regulatory sequences represented by SEQ ID No: 2 or a mutant thereof, at least one marker gene for positive clone selection, optionally two recognition sequences for a recombinase flanking the marker gene, two sequences for homologous recombination (homologous arms) flanking the marker gene and the target gene and optionally at least one marker gene for negative clone selection flanking the homologous arms.

As marker gene for positive clone selection every gene can be used that, under certain selective conditions, only allow the carrier of this gene to survive. Preferably the selection marker is an antibiotic resistance gene, most preferred the gene is the neomycin resistance gene. Positive selection markers are needed to identify the ES cells which contain the recombinant vector.

As marker gene for negative clone selection every gene can be used that is translated into a toxic protein for normal ES cell. Preferably the selection marker is a gene coding for a toxin, for instance diphteria toxin, alpha-toxin, streptozotocin or botulinum toxin, preferably diphteria toxin. Further genes can be used as negative selection markers which stop the cell cycle under certain selective conditions. For instance by terminating the DNA replication using nucleotide base analogues due to thymidine kinase activity.

Negative selection markers are used to select ES cells with the correct homologues recombination. Since both toxic genes are located outside the homologous arms, upon correct recombination they do not join the TNF-α containing segment of the mouse genome which is being replaced by the human part of the construct. Thus, the fragments containing the negative selection genes do not integrate into the genome and are quickly lost during cell division. To the contrary, when a non-homologous integration event occurs, the linearized vector usually integrates as a whole, together with the negative selection cassettes. In such case, toxic selection genes are transcribed and translated, which creates selective disadvantage for clones with non-homologous integration. Since non-homologous integration is much more likely than homologous recombination, negative selection is vital for success of a gene targeting experiment.

Usually thymidine kinase is used alone in gene targeting experiments in order to stop proliferation of ES cells with a non-homologous integration of the target gene. Theoretically, these cells would die and the ES cells with a homologous integration of the target gene would proliferate. In practice, many of them survive for various reasons. Thus, a second marker for negative selection can be used in the recombinant vector. The second marker codes for a protein which is toxic by itself. Thus, the negative selection marker is active without any special culture conditions. The proportion of clones with homologous recombination can be significantly increased using a second negative selection marker.

In a preferred embodiment the recombinant vector according to the invention is a vector, wherein the at least one marker gene for positive clone selection is an antibiotic resistance gene, preferably the gene coding for neomycin resistance and at least one marker gene for negative clone selection is a gene coding for a toxin, preferably for diphteria toxin or a gene coding for thymidine kinase.
As recombinant vector any known recombinant vector can be used which is able to integrate the appropriate amount of base pairs. As a preferred recombinant vector a pBluescriptKS(+), also known as pBSKS(+) vector is used for generation of a transgenic mouse according to the invention.

The two recognition sequences for a recombinase flanking the marker gene are preferably loxP sites. The Cre recombinase stems from the E. coli bacteriophage P1 and mediates the site-specific recombination between two identical loxp motives in an intramolecular or intermolecular manner. The recombinase Cre of the E. coli bacteriophage P1 is a site-specific recombinase mediating a DNA reorganization via its DNA target sequence, namely loxP. The loxP sequences consist of an 8 bp spacer region flanked by two 13 bp inverted repeats, which serve as recognition sequences for the DNA binding of Cre. The recombination event is only dependent on these two components and is conducted with absolute reliability. It was appreciated that the Cre-loxP system effectively catalyses recombination events in prokaryotic as well as in eukaryotic cells including those from yeast, plants, insects and mammals. Site-specific recombination systems are used to a large extent as tools for conditional genetic changes in single cells and animals. In the case of an excision the region of a DNA sequence between two loxP recognition sequences is cut out.

There is a multiplicity of further site-specific recombination systems based on a two-component system that may be used in the present case. All such systems have in common specific repeating DNA sequences. Each of these sequences consists of two recognition sequences that are separated by a spacer and are inversely repetitive to each other. The two components are identical in this case. Further examples are the Flp-FRT system of S. cerevisiae, the zygosaccharomyces rouxii pSR1, the resolvase-rfsF and the phage Mu Gin recombinase system.

According to the invention the homologous arms for homologous recombination are located on both sides of the target gene and the positive selection marker on the recombinant vector. The sequences chosen for homologues recombination are located on both sides of the target gene in the mouse genome. The particular sequences for the left and right arms are chosen based on homology map between mouse and human TNFα loci to make sure that no insertion or duplication of homologous mouse sequences occurs when merging human TNFα gene into mouse genome. According to the invention the genes located next to the TNFα gene, in particular the sequences of the murine lymphotoxin α and lymphotoxin β genes are used as left and right arms for homologous recombination. The recombination events occur somewhere within the left and right arms. Since recombination occurs with nucleotide precision, no damage is done to either of these two murine genes. Thus, the human TNFα knock-in mouse of the invention does not have any genetic damage due to the genetic modification.

In a preferred embodiment homologous arms correspond to nucleotides 21523738-21528888 (left arm) and 21533435-21538973 (right arm) of Mus musculus chromosome 17 genomic contig, strain C57BL/6J, accession NT_039649.7, GI:149268951.

The components of the vector were cloned into the vector backbone using the standard protocols of molecular biology known to the person skilled in the art (T. Maniatis, J.F. Sambrook. Molecular Cloning: A Laboratory Manual. 1989, CSHL Press). Thereby the position of the components and the orientation of the DNA fragments has to be considered. Correct orientation and position of the components were confirmed by digests with restriction enzymes and DNA sequencing. Digests and DNA sequencing can be done according to the known standard protocols (T. Maniatis, J.F. Sambrook. Molecular Cloning: A Laboratory Manual. 1989, CSHL Press).

The recombinant vector can be linearized with an appropriate enzyme. Every enzyme can be used according to the invention which restriction site is located once on the recombinant vector and which leave the targeting insert intact and connected to both negative selection cassettes. Preferably the recombinant vector can be linearized using Clal. The linearized vector is transfected into the ES cells. Any known method of transfection, for instance calcium phosphate, lipofection, nucleofection, heat shock, electroporation, gene gun, microinjection or magnetofection can be used according to the invention. Preferably electroporation is used for transfection of the recombinant vector.

Suitable ES cells according to the invention are any murine ES cells. Preferably murine ES cells are used which are robust and show high transfection and homologous recombination rates. In particular murine ES cells are used which are of C57BI6 origin, most preferred Bruce 4 cells. This has an advantage for immunological research because the transgenic mice produced are immediately on the "clean" genetic background.

The present invention further relates to embryonic stem cells, comprising a recombinant vector according to the invention. In a preferred embodiment the ES cells have been transfected with a vector according to the invention, in particular transfected with a vector according to the invention. In another preferred embodiment ES cells of the same genetic background, as the recipient mouse, in particular Bruce 4 cells are used. That means that the ES cells and the recipient mice belong to the same mouse strain. Thus, a genetically modified mouse can be produced on a clean and preferred genetic background, such as C57BI6. Preferably, the invention relates to murine ES cells wherein the target gene of the vector was introduced into the mouse genome due to homologous recombination.

ES clones with correct homologous recombination were selected by combining the culture conditions specific for positive and negative selection. Positive ES cell clones were further characterized by genomic Southern analysis, PCR and DNA sequencing. Characterization experiments were carried out due to standard protocols known by the person skilled in the art (T. Maniatis, J.F. Sambrook. Molecular Cloning: A Laboratory Manual. 1989, CSHL Press).

Subsequently, the ES cells containing the human TNFα gene at the correct position have to be injected into a blastocyst of a C57BI6 mouse (3 to 4 days after fertilization) in order to obtain chimerae. The blastocyst containing about 100 cells is implanted in pseudo-pregnant females. Blastocyst injection and implantation were done according to standard protocols known by the person skilled in the art. (For reference see for instance Tessarollo L., Manipulating mouse embryonic stem cells. Methods Mol Biol. 2001;158:47-63 or Bonin A., Reid SW, Tessarollo L. Isolation, microinjection, and transfer of mouse blastocysts. Methods Mol Biol. 2001;158:121-34.). Usually albino mice are used as recipient mice, if ES cells from black mice are implanted in order to identify the chimeric offspring by mixed colour. In another embodiment the blastocyst can be implanted in normal, i.e. black C57BI6 mice. The offspring is genotyped for the transgene to identify the chimeric animals.

Embryonic stem cells of the mouse are capable of participating in all aspects of the development including the germ track. Therefore, the injected ES cells can also become germ cells, which then transmit the human TNFα gene. The developing mice are chimerae with respect to the changed gene, hence some of the tissues derive themselves from the injected ES cells, others from the normal blastocyst. Thus, the offspring of the mouse has to be PCR typed or analysed by Southern blotting for the expected recombination event, i.e. the human TNFα gene until the germline transmission of the complex genomic modification is achieved. PCR typing and Southern blotting can be done according to standard protocols known by the person skilled in the art (T. Maniatis, J.F. Sambrook. Molecular Cloning: A Laboratory Manual. 1989, CSHL Press).

In particular a real clean knock-in, i.e. one gene was taken out, another gene was put on its place should be achieved. Thus, the traces of genetic engineering have to be removed. Neo selection marker which is absolutely needed at the ES stage has to be deleted, either in vitro or in vivo. Preferably the Neo selection marker is deleted in the correct ES clone after homologous recombination, for instance by transfecting the cells with another vector comprising the Cre recombinase. Most preferred the Neo selection marker is deleted after successful germ-line transmission of the mutation by crossing the knock-in mice with a Cre-deleter transgenic mice in which Cre is expressed in many or most of cell types, in particular beta-globin-Cre transgenic mice can be used. After this procedure the Cre-transgene has to be removed itself. Preferably the human TNFα Knock-in mice are intercrossed until the Cre transgene is crossed-out and a clean homozygous human TNFα Knock-In mouse is obtained.

It is another object of the invention to provide a in-vivo method for screening, investigating and/or preclinical testing of compounds and/or drug candidates for human anti-TNFα therapy comprising the following steps:
a) providing a transgenic mouse according to the invention
b) inducing a pathogenic TNF-α response in said mouse
c) treating the mouse of step b) with the compounds and/or drug candidates to be tested
d) analysing the human anti-TNFα potential of said compounds and/or drug candidates administered in step c) by evaluating the changes in animal physiology and/or by comparing the changes in animal physiology with control animals
e) optionally repeating steps c) and d) with an appropriately modified form of the compounds and/or drug candidates to be tested.

In particular the human TNFα -Knock-in mice according to the invention are used as an in-vivo animal model for screening, investigating and/or preclinical testing of compounds and/or drug candidates which are effective for human anti-TNFα therapy. Recent study (Anolik, J.H. et al 2008;. J Immunol 180: 688-692) has clearly indicated that the consequences of TNFα blockage in patients with regard to organization of lymphoid tissues are the same as in mice, based on earlier knock-out and blockade studies (Tracy D. et al. 2008, Pharmacol. Ther. 117: 244-479). Thus a humanized TNFα Knock-In mouse is a good preclinical and clinical animal model to investigate the effects of anti-TNFα therapy.

In particular compounds, drugs and/or drug candidates are screened and/or investigated for the ability to effect the properties of human TNFα in said transgenic mice of the invention. According to the invention, properties of human TNFα are any characteristics which belong to the TNFα physiological properties, for instance stability, biological activity, distribution in the organism, degradation or inhibition.

In another preferred embodiment compounds which are known to be effective or newly identified compounds in human anti-TNFα therapy can be compared with respect to their properties in order to make human anti-TNFα therapy more specific to distinct diseases and/or the individual needs of various types of patients. According to the invention, properties of the compounds are any characteristics which belong to the biophysical and/or pharmacological properties, for instance stability, degradation, distribution in the target organism, pharmacological kinetic, efficacy, therapeutic mechanism and/or alleviation or elimination of the symptoms.

In a preferred embodiment of the invention therapeutically useful compounds for human anti-TNFα therapy are found to treat human inflammatory diseases in which TNFα plays a key role. In particular human anti-TNFα therapy means the treatment of autoimmune disorders such as rheumatoid arthritis (RA), ankylosing spondilitis (AS), inflammatory bowel disease (IBD), acute and chronic immune diseases associated with transplantation, psoriasis, juvenile arthritis, some forms of cancer and others.

In a preferred embodiment of the in-vivo method for screening, investigating and/or preclinical testing of compounds and/or drug candidates for human anti-TNFα therapy LPS-Dgal is injected into said mouse in step b). Thus, acute liver toxicity is induced in said mice. This is a very simple method to induce acute pathological TNFα activity in mice. According to the invention every other method of inducing acute pathological TNFα activity can be used as well, for instance injection of SEB/Dgal or ConA. Untreated mice with acute liver toxicity become moribund within 6 - 10 hours.

In another preferred embodiment of the in-vivo method for screening, investigating and/or preclinical testing of compounds and/or drug candidates for human anti-TNFα therapy in step c) human TNF-α blocking compounds, preferably Infliximab, Adalimumab, Etanercept, mutant TNFα capable of disrupting existing TNFα trimers, dominant negative mutants and/or small molecules which would interfere with TNF-TNF receptor are injected into a mouse according to the invention. Dominant negative mutants which can be injected as human TNF-α blocking compounds in step c) of said in-vivo method are described in Science 2003 301 (5641):1895-1898.

In another preferred embodiment of the in-vivo method for screening, investigating and/or preclinical testing of compounds and/or drug candidates for human anti-TNFα therapy the TNF-α plasma levels are analysed and/or the mortality rate of the mice according to the invention is detected and/or compared to healthy control mice in step d). Further physiological parameters of the transgenic mice of the invention can be investigated, for instance damage to lymphoid organs, changes in gene expression, in particular of the TNFα gene and every other gene belonging to the immune system and/or any other physiological parameter referring to the inflammatory response, such as fever, local cytokine production, acute phase proteins or liver enzymes.

It is another object of the invention to provide an in-vitro method of screening, investigating and/or preclinical testing of compounds and/or drug candidates for human anti-TNFα therapy wherein the cells, tissues and/or cell lines derived of a mouse according to the invention are used.

In a preferred embodiment the method comprises the steps of
a) deriving cells, cell lines and/or tissues from a transgenic mouse according to the invention and/or using cells, cell lines and/or tissues according to the invention
b) cultivating said cells, cell lines and/or tissues in-vitro
c) inducing a toxic TNF-α response in said cells, cell lines and/or tissues and/or adding human TNF-α to said cells, cell lines and/or tissues
d) adding human TNF-α blocking compounds, in particular Infliximab, Adalimumab, Etanercept, mutant TNFα capable of disrupting existing TNFα trimers, dominant negative mutants and/or small molecules which would interfere with TNF-TNF receptor to said cells, cell lines and/or tissues
e) analysing the TNF-α level and/or marker for cytotoxicity compared to control cells, cell lines and/or tissues
f) optionally repeating steps d) and e) with an appropriately modified form of the test compound.

The present invention further relates to a compound developed using a method according to the invention. In particular the invention relates to compounds, drug candidates and/or prodrugs identified and/or developed using an in-vivo or in-vitro method based on the human TNFα Knock-In mouse according to the invention and/or cells, tissues and/or cell lines derived thereof.

The compound, drug candidate and/or prodrug is not particularly limited, but examples thereof include peptides, proteins, in particular antibodies and/or protein receptors, non-peptide compounds, synthetic compounds, fermented products, cell extracts, inorganic compounds and/or organic compounds. With an in-vivo human TNFα animal model of the invention it is possible to compare the efficacy of various known and/or novel TNFα related drugs to each other, to understand the mechanisms of action of various TNFα related drugs which are in clinical use, for instance why Infliximab is active in Crohn's disease patients and Etanercept is not, minimizing the side effects of anti-TNFα therapy by sparing beneficial physiological functions of TNFα, such as maintenance of bactericidal granulomas in protection against Mycobacteria. It is further possible to understand the mechanisms of action of TNFα in TNFα related diseases. In

summary, it is an advantage of the invention to model TNFα-mediated diseases in mice and investigate and/or treat them with the human therapies in order to test the various novel therapies. Without "humanized" mice such experiment would not be possible.

Short description of the figures
Fig. 1 shows the pBSKS(+) recombinant vector used for homologous recombination.
Fig. 2 shows the different stages of the gene Knock-In procedure
Fig. 3 shows the analysis strategy of the transgenic mice using Southern Blotting.
Fig. 4 shows the results of genomic Southern Blot analysis after Asp718l and BamHI digest, respectively.
Fig. 5 shows the results of genomic PCR analysis using primers specific for the right junction between human TNFα gene and mouse locus.
Fig. 6 shows the efficacy of Infliximab in the human TNFα-Knock-In mouse after induction of acute liver toxicity.

The present invention is explained in greater detail by the following examples. These examples are illustrative of the present invention and are not to be construed as limiting thereof.

### Example 1: Tissue culture of feeder cells

Tissue culture dishes for ES cell culture has to be prepared with feeder cells which provide a LIF enriched environment for the ES cells. The ES cells are grown on top of these feeder cells. Primary mouse embryo fibroblasts (PMEFs) are prepared directly before use from healthy C57BI6 mice (Tessarollo L. Manipulating mouse embryonic stem cells. Methods Mol Biol. 2001;158:47-63); PMEFs that are treated with mitomycin C (MMC, Sigma # M0503) are called feeders or feeder layer. For MMC solution 5ml DPBS (Invitrogen # 14190-169) were added to the 2mg vial of MMC using a syringe. The 5ml were added to 195ml of DMEM [1000 ml bottle of DMEM (4.5g/L glucose, with L-Glutamine, without Sodium Pyruvate, Chemicon # SLM-121-A), 1ml 0.1M Betamercaptoethanol (BME; 70µl of 14.3 M BME, Sigma # M7522, in 10mls of DPBS, filtered through a 0.2µM filter and stored at 4°C), 5ml 10,000units Penicillin; 10mg/ml Streptomycin (Invitrogen # 15140-122)] + 10% FBS (Invitrogen # 10439-024) (D10%) or DMEM + 15 % FBS (D15%). The medium was filtered through 0.2µM filter and transferred at 12 ml each to 15ml tubes and frozen at -20°C.

To prepare a 100mm feeder dish a confluent 150mm PMEF dish was obtained. Medium was aspirated and cells were treated with 5-7 ml MMC solution for 2-4 hours at 37°C. After incubation the cells were washed with DPBS three times to remove all MMC. 5 ml trypsin/EDTA (Trypsin/EDTA (Invitrogen # 25200-056) + 1% sterile filtered chicken serum (Invitrogen # 16110-082) were added to the cells and the cells were incubated at room temperature until the PMEFs detach from bottom of the dish. 20mls of DMEM + 10 % FBS (D10%) were added to each dish and cells were collected into a 50ml tube. Cells were counted with a hemocytometer and calculated in cells/ml. 3x10⁶ feeders were seeded into one 100mm dish in a total of 10ml D10%. After PMEFS are MMC treated they are incubated for 1-1.5 weeks. MTK-Neomycin 2 (Neo2) PMEFS are prepared equally to PMEFs from any neomycin-resistant source, for instance any classical KO mouse. Usually MTK-Neomycin 2 (Neo2) PMEFS are made from classical LT-alpha knockout mouse (De Togni et al, Science 1994, 264(5159):703-707). Neo2 PMEFs are treated equally to PMEFs and can be cultured up to passage number 5. D10% is used for PMEFs alone and D15% is used if ES cells are cultured on feeders.

### Example 2: Tissue Culture of ES cells

Bruce 4 cells (directly received from their author Colin Stewart, Singapore) are cultured in D15% on a feeder layer. One vial (about 3 million cells) are cultured in the 100mm feeder dish, in a total volume of 10ml D15%. They will be confluent in 3-4 days. ES cells need to be trypsinized within 4-6 days after seeding or when confluent, preferably within 1-2 days after confluency or they will begin to differentiate. For trypsinyzing warm DPBS, trypsin/EDTA are used. Media in should be warmed to 37°C in water bath before use.

### Example 3: Preparation of the recombinant vector

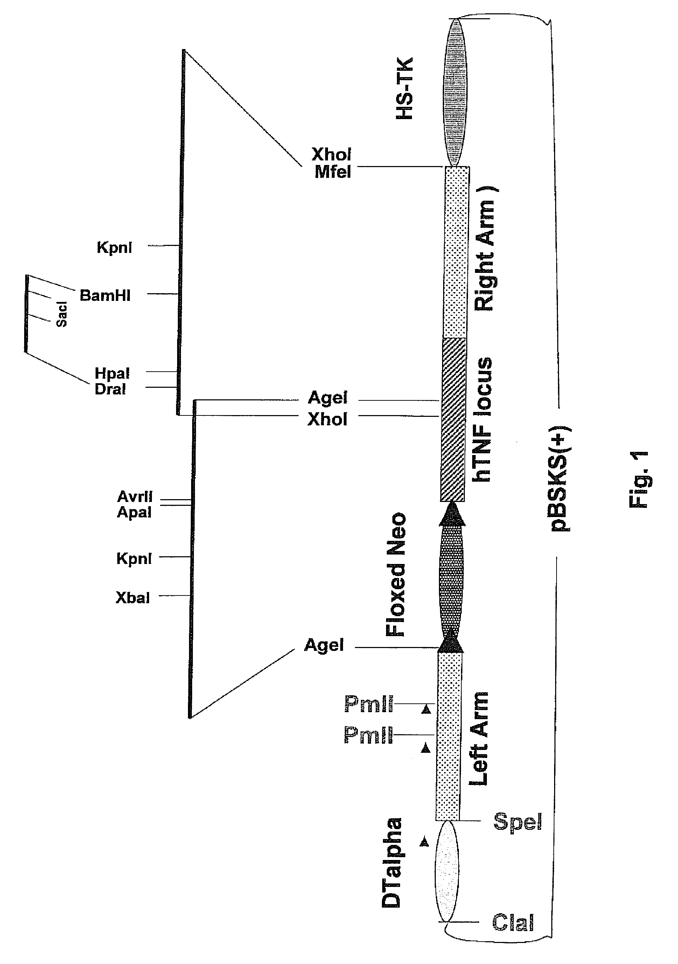

The recombinant vector is shown in Fig 1 and is generated using standard cloning protocols (T. Maniatis, J.F. Sambrook. Molecular Cloning: A Laboratory Manual. 1989, CSHL Press). The recombinant vector is based on a pBSKS(+) backbone (Stratagene). The negative selection markers diphteria toxin alpha (DTalpha) and Herpes Simplex thymidine kinase (HS-TK) are located at both ends of the insert and outside the homologous recombination arms (left arm and right arm). Downstream to the DTalpha cassette (SEQ ID NO 12; 1.2 kb) the left arm (SEQ ID NO 3; 5.1 kb) is localised, followed by the positive selection marker, the neo cassette coding for a neomycin resistance (SEQ ID NO, 4; Floxed Neo, 2.2kb). After the neo cassette the human TNFα gene (SEQ ID NO 2; hTNF locus, 4.1kb) is cloned, followed by the right arm (SEQ ID NO 5; 5.3 kb). The cloning of the insert is completed by adding the second negative marker the HS-TK cassette (SEQ ID NO 6; 2.9 kb). The vector can be linearized upstream of the DTalpha gene with the restriction enzyme Clal. The position of further restriction sites are marked together with the name of the appropriate digesting enzymes. These sites can be used for control digests of the recombinant vector. The final vector was fully sequenced using standard sequencing service.

### Example 4: Transfection of the recombinant vector

A 100mm dish of ES cells is grown until confluency as shown in example 2. 1-3 days before electroporation, six 60mm feeder dishes are prepared by MMC treatment, washed, trypsinized, counted and seeded with 8 x 10⁵ feeders/dish in a total of 5ml D15% in six 60mm dishes as shown in example 1.

For electroporation the recombinant vector of example 3 is linearized with Clal (New England Biolabs # R0197). The linearized DNA is purified with phenolchloroform (Fluka # 77617), chloroform (Sigma # C2432), precipitated with NaCl (Carl Roth # 3957.2) and 100% ethanol (Carl Roth # 9065.2) and washed with 70% ethanol. Cleaned DNA is resuspended in TE (Part of Qiagen # 12362) or dH₂O at a concentration of 1µg/µl. Each electroporation uses 30µg DNA.

For electroporation the media from the six 60mm feeder dishes is aspirated and 4ml of D15% are added to each dish. Cells are re-incubated at 37°C. The media from the ES cells in the confluent 100mm dish is aspirated as well and cells are rinsed with DPBS. Afterwards 5ml of trypsin/EDTA are added and ES cells are incubated at room temperature until cells are detached from the bottom of the dish and individual ES cells can be detected. 25ml of D15% are added, cells are collected in a 50ml tube, counted with a hemocytometer and calculated as cells/ml. 1-3 x 10⁷ ES cells are needed for one electroporation. The feeders do not have to be excluded in the cell counts because there are not enough feeder cells to make a difference. 1-3 x10⁷ cells are added to one 15ml tube, centrifuged at 1000rpm for 5 minutes, rinsed with DPBS once and spinned again. The procedure is repeated for a total of three PBS washes. 6 x 10⁶ cells are treated equally and resuspended in 1 ml of D15%. These cells are added to one of the 60mm feeder dishes and are used as control cells. They will be G418 sensitive.

To the 1-3 x 10⁷ cells for electroporation 0.8ml of ice cold DPBS are added and cells are transferred to an electroporation cuvette w/ 0.4cm gap. 30 µg (30µl) of recombinant vector are added to the electroporation tube and mixed well with a 1ml pipette without making bubbles. The electroporation machine is set to 250µF, 320 volts. The electroporation cuvette is put into the chamber with notch on the outside and electroporated. The electroporated cells are added immediately to 4.5ml of D15% and given at 1ml each to the remaining five feeder dishes.

### Example 5: Tissue culture under selection conditions

Media are aspirated the one day after electroporation and all six dishes of Example 4 are treated with D15% containing 350µg/ml G418 (Invitrogen # 10131-019; 20 ml of 50 mg/ml solution). Only cells carrying the positive selection marker, in particular the neomycin resistance, will survive in the G418 media. The non-electroporated control cells shall die. Further the media contains 2 µM ganciclovir (Sigma # G2536-100MG) for negative selection using the thymidine kinase. In cells with non-homologous recombination the DNA replication will be terminated due to incorporation of ganciclovir. As the diphteria toxin alpha is toxic by itself no special culture conditions are needed for functional negative selection. Cells are re-feed in 48 to 72 hours later or when cells begin to die. Cells can be re-feed every day if desired. Positive targeted clones should be picked after 6-8 days.

### Example 6: Picking of G418 resistant clones

Using a dissecting microscope undifferentiated clones are picked with a pipet tip outfitted on a mouth pipette by scraping the clone up with the tip while drawing it into the tip using the mouth pipet. After picking a clone is expelled into one well of a 48 well feeders plate prepared as shown in example 1. Approximately 20 G418 resistant ES clones from each of the five 60mm dishes of example 4 cultured as shown in example 5 are picked. Optionally the clone can be trypsinized before adding to the 48 well by adding each clone to a well of a 96 well plate that has 100µl of trypsin/EDTA added and incubating at room temperature for a few minutes. After incubation the clones are broken up by triturating with a P-200 pipet. The dissociated clones are added to a well of the 48 well feeder plate. Single clones are grown to confluency as shown in example 2, used for DNA preparation or trypsinized and frozen by -80°C in 350µl freezing media (D15% with a final concentration of 7% DMSO (Sigma # D4540)).

### Example 7: ES Cell lysis and DNA preparation

600µl of lysis buffer (50mM Tris-HCl (Carl Roth # 4855.3 and # 9277.1 respectively); 100mM EDTA (Sigma # E5134), pH8; 100mM NaCl; 1% SDS (Sigma # L4390) with 420 µg/ml of proteinase K (Biodeal # CH1005,0200) are directly added to each well of a 48 well plate. Cells are incubated at 37°C overnight, shaking is not necessary. Lysed cells can be stored at this point by - 20°C.

For DNA preparation the lysed cells are transferred to 1.5 ml eppendorf tubes and mixed on an eppendorf shaker for 5 minutes to get to room temperature. All the following steps are done at room temperature. 200µl of saturated NaCl (approximately 6M (Carl Roth # 3957.2) are added to each tube and mixed again for 5 minutes on the eppendorf shaker. Then the tubes are spun for 10 minutes in an eppendorf centrifuge at 14k rpm. The 600 µl supernatant are collected, transferred to a new tube and 500µl of isopropanol (Sigma # I9516) are added to precipitate the DNA. The precipated DNA is spun at 14K rpm for 5 minutes, washed twice with 500µl of 70% EtOH and dried. Pellets are resuspended in 100µl TE buffer.

### Example 8: DNA preparation from the transgenic mice

For genotyping of the transgenic mice a short piece of an ear or the tail are cut and transferred into an eppendorf vial containing 600µl of lysis buffer (example 7) with 420 µg/ml of proteinase K. The tissue is incubated at 37°C overnight under continuous shaking. DNA preparation was done as shown in Example 7.

### Example 9: Stages of gene replacement

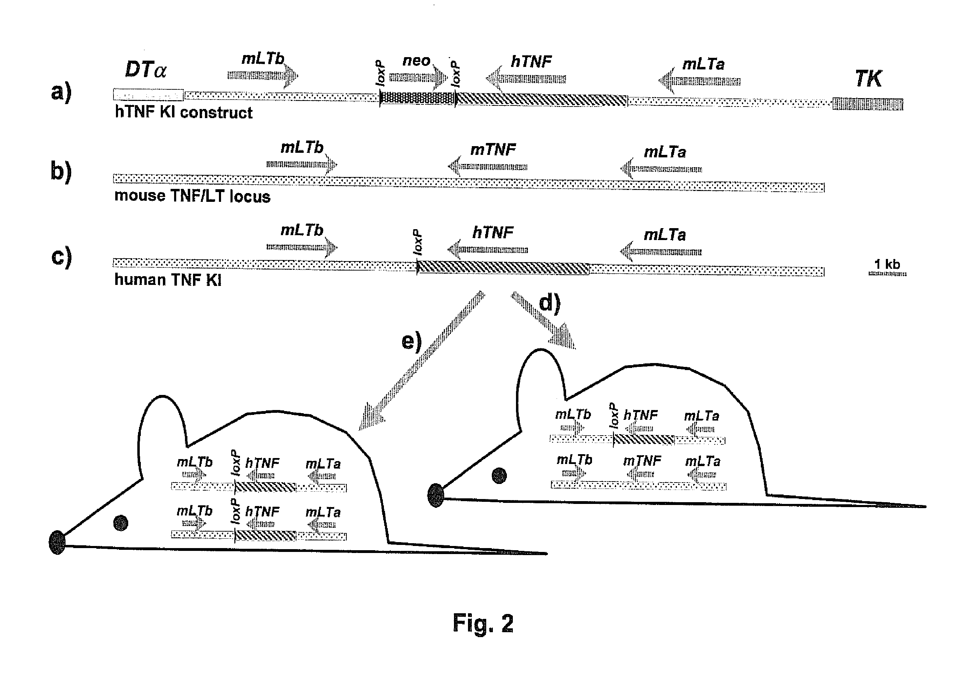

Fig. 2 shows the stages of gene Knock-in before and after homologous recombination and deletion of the Neo cassette. Only one allele is shown. Before the experiment Bruce 4 cells contain two alleles with the murine TNFα gene as shown in Fig. 2b. After transfection the recombinant vector shown in Fig. 2a is localized in the cytosol in addition to the murine TNFα gene (Fig. 2b). After the recombination event and the deletion of the Neo cassette the human TNFα gene replaces the murine homologous. The human TNFα KI is shown in Fig. 2c. The only trace of the gene modification is the loxP site located at the end of the human TNFα gene. A mouse having one allele shown in Fig. 2b and one allele shown in Fig. 2c would be heterozygous for the gene modification (Fig. 2d). Such a mouse produces both the human and the murine TNFα. A homozygous mouse (Fig. 2e) having both alleles modified as shown in Fig. 2c. This mouse is a real humanized TNFα mouse with no endogenous murine TNFα.

### Example 10: Genotyping of the ES cells and the transgenic mice by Southern Blotting

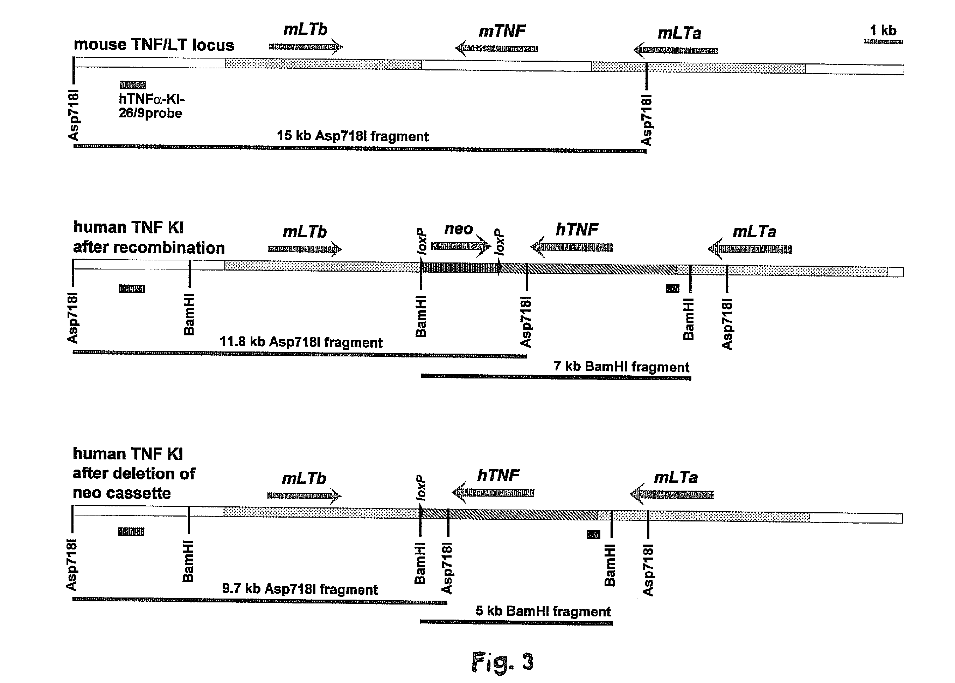

The extracted DNA of examples 7 and 8 was digested with different restriction enzymes for Southern blotting. Fig. 3 shows the enzymes used and the expected DNA fragments using appropriate hybridization probes for identifying the different configurations of the TNFα-locus. Using Asp718I (Roche) a 15 kb fragment is expected for the wild type mouse TNF/LT locus. After successful recombination the size of the detected fragment is 11.8 kb and the size of the fragment after deletion of the neo cassette is 9.7 kb. Using Bam HI (New England Biolabs) a 7 kb fragment is expected for the human TNFα-KI after recombination and a 5 kb fragment is expected after deletion of the neo marker. After digest with either Asp718I or Bam HI the DNA fragments were electrophoresed on an agarose gel to separate them by size. After separation the DNA was denatured and transferred to a nitrocellulose membrane Hybond-N (Amersham Biosciences # RPN303N) using the upward capillary transfer method. Digest, electrophoresis and Southern blotting were done according to standard protocols known to the skilled person (Brown T. Analysis of DNA sequences by blotting and hybridization. Current Protocols in Molecular Biology (1999) 2.9.1-2.9.20, John Wiley & Sons, Inc). The membrane was hybridized with either the hTNFα-KI-26/9 DNA probe (SEQ ID NO 13) for Asp718I digest or with hTNFα-KI-BamHI probe (SEQ ID NO 14) for BamHI digest.

### Example 11: Results of Genotyping

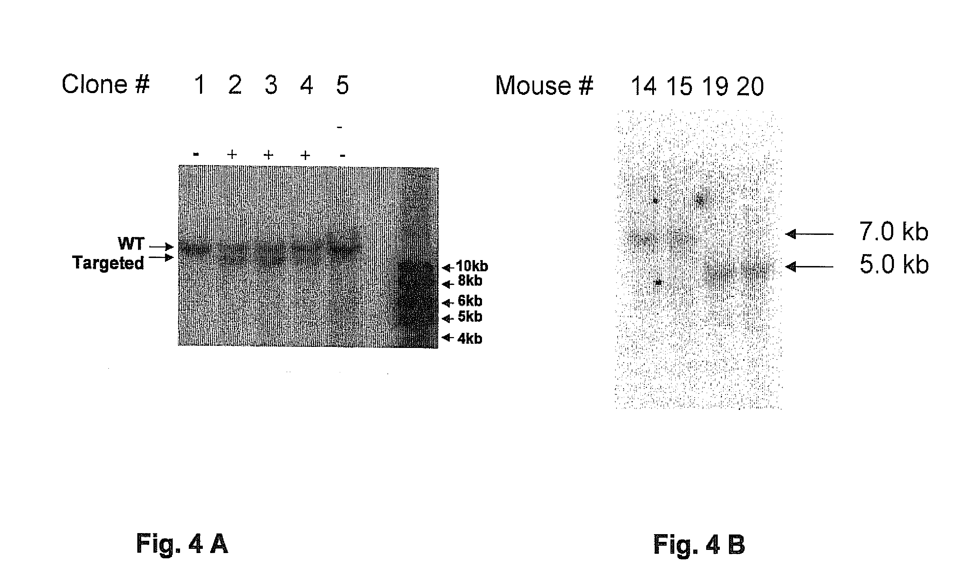

DNA from ES cells was extracted as shown in example 7 and genotyped according to example 10. Digest was performed with Asp718I. Fig. 4A shows the results after hybridisation with human TNFα-KI-26/9 DNA probe. Clone 1 and 5 show a single band at 15 kb. This band is specific for the mouse TNFα wild type allele. In these clones the recombination event has not happened successfully. Clones 2, 3 and 4 show one band at 15 kb and one band at 11.8 kb. The shorter band is specific for the human TNFα KI after recombination. Thus, clones 2, 3 and 4 are heterozygous for the required mutation. They contain one allele with the mouse TNFα and one allele with the human TNFα. These clones are selected for blastocyst injection.

DNA from four mice #14, #15, #19 and #20 was extracted as shown in example 8 and genotyped according to example 10. Digest was performed with BamHI. Fig. 4B shows the results after hybridisation with hTNFα-KI-BamHI probe. Mouse #14 and #15 show a single band at 7.0 kb. This band is specific for mice that carry the human TNFα gene and still the neo-selection marker (neo+). After the Cre mediated recombination event the neo gene is cut out. Thus, the 7.0 kb band (neo+) is absent in mouse #19 and #20 and a shorter band of 5.0 kb (neo-) appeared. The mouse #19 and #20 show the genotype desired according to the invention.

### Example 12: Genotyping of the ES cells and the transgenic mice by PCR

The genetic modification was confirmed by identifying the two junctions between mouse and human sequences that did not exist before the genetic modification. As shown in Example 9 the loxP site is still located at the end of the human TNFα gene after the effective gene knock-in. The sequence of the loxP site is shown in SEQ ID NO 7 and can be used as marker for effective gene knock-in using PCR.

Upstream of the introduced human TNFα gene a human/mouse right junction fragment occurs. This fragment is combined of human and mouse parts and several nucleotides of the cloning junction between the mouse and human parts The sequence is shown in SEQ ID NO 8. This fragment can be amplified by PCR as a marker for effective gene knock-in. As the fragment does not occur in nature the PCR does not work on either wild type mouse or wild type human DNA. PCR was done under following conditions. Initial degeneration for 3 min at 94°C, followed by 35 cycles of degeneration at 94°C for 30 sec, annealing at 60°C for 30 sec and amplification at 72°C for 30 sec; and final amplification for 5 min at 72°C.

The following three primers are being used in one PCR reaction:
hTNF_kn_in_1: 5'-ATGTACCGCAGTCAAGATATG (SEQ ID NO 9)
hTNFkn_in_2: 5'-TTGAGTCCTGAGGCCTGTGTT (SEQ ID NO 10)
hTNFkn_in_3: 5'-TGTTGTATAGGACCCTGAGAA (SEQ ID NO 11)

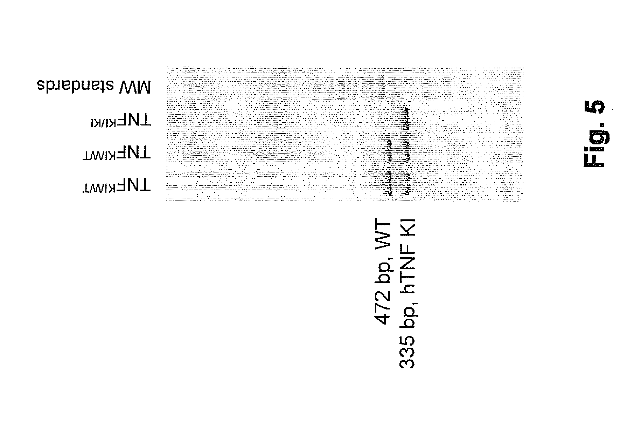

Primers 1 and 3 amplify a control mouse fragment of 472 bp which is absent in a mouse with a homozygous Knock-In mutation, while primers 1 and 2 amplify the unique human/mouse right junction fragment of 335 bp. In heterozygous mice and/or cells both fragments are amplified. In homozygous human TNFα KI mice and/or cells only the 335 bp fragment can be amplified. Figure 5 shows the results. In the first and second lane both fragments appear. These mice are heterozygous for the mutation. Lane three shows only a single band by 335 bp identifying a homozygous genotyp. Lane four shows a molecular weight marker (Fermentas SM0322).

### Example 13: Test of the TNFα Knock-In mice as animal in vivo model for human anti-TNFα therapy

For testing the efficacy of human anti-TNFα drugs, in particular Infliximab, acute liver toxicity was induced in mice by injecting intraperitoneal (i.p.) LPS (Sigma # L3024, 10mcg/mouse) and D-Gal (Sigma # G1639, 20mg/mouse). TNFα blockers (2mg/mouse) against human TNFα (Infliximab) or against mouse TNFα (anti-mTNFα antibody) were injected i.p. 30 min later. Survival rate was monitored during 48 hours after LPS/D-Gal injection as a marker for efficacy of the injected TNFα blocker. Moribund mice were humanely sacrificed. For control wild type (WT) mice and TNFα knock-out (TNF KO) mice were treated accordingly. Tests were performed with three mice per group.

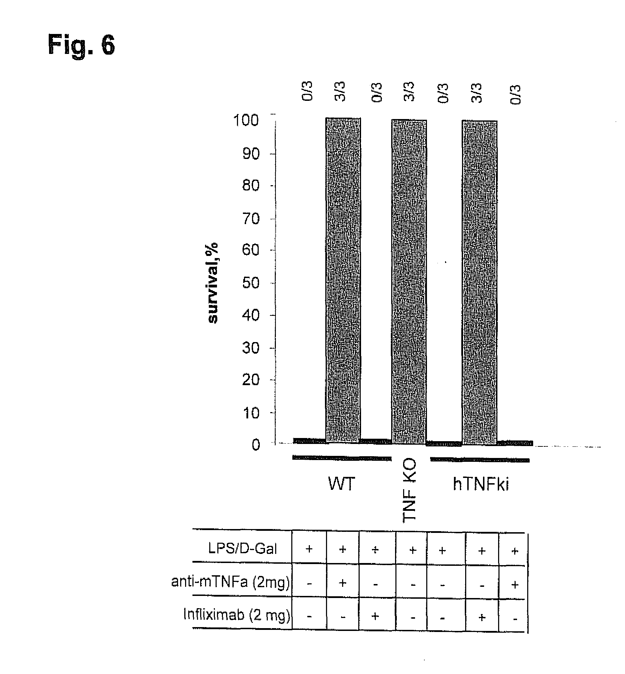

Figure 6 shows the result of these tests. WT mice are injected with LPS/D-Gal alone, with LPS/D-Gal + anti-mTNFα antibody and with LPS/D-Gal + Infliximab. All animals died, if LPS/D-Gal is injected alone due to acute liver toxicity. Animals which were injected with the anti-mTNFα antibody survived because the TNFα activity was neutralized by the mouse antibody. Infliximab does not work in WT mice, so that all animals died. TNF KO mice were injected with LPS/D-Gal alone, but acute liver toxicity could not be induced due to the complete knock out of TNFα. Thus, all animals survived. Humanized knock-in mice according to the invention are injected as the WT mice. Mice treated with Infliximab survived and mice treated with the anti-mTNFα antibody died. It can be seen that Infliximab only works in "humanized" knock-in mice according to the invention and the anti-mTNFα antibody only works in wild-type mice and have no effect in humanized knockin mice. Thus, the humanized TNFα Knock- in mouse according to the invention can be used as an excellent animal model for testing of human anti TNFα therapy.

## Claims

1. A transgenic mouse, wherein the complete endogenous murine TNFα-gene including its upstream regulatory sequences represented by SEQ ID No: 1 is replaced by the human homologous TNFα-gene including its upstream regulatory sequences represented by SEQ ID No: 2 or a mutant thereof, wherein the mutant is **characterized by** deletion, addition, substitution, and/or inversion of one or more nucleotides so that the functional properties of the modified human TNFα-gene are conserved.

2. The transgenic mouse of claim 1, wherein the transgenic mutation is present in somatic and germ cells.

3. The transgenic mouse of claims 1 or 2, wherein said transgenic mouse is vital, fertile and capable of transmitting the transgenic modification to its offspring.

4. The transgenic mouse of anyone of claims 1 to 3, wherein the mouse is homozygous for the transgenic mutation and/or wherein the TNF-α functions are mediated completely by the genetically introduced human TNF-α.

5. The transgenic mouse of anyone of claims 1 to 4, wherein the mouse is a C57BI6 mouse.

6. Cells, tissues and/or cell lines derived from the transgenic mouse of anyone of claims 1 to 5.

7. A recombinant vector for homologous recombination in murine embryonic stem cells comprising the genomic and/or cDNA sequence encoding the human TNFα-gene including its upstream regulatory sequences represented by SEQ ID No: 2 or a mutant thereof, at least one marker gene for positive clone selection, optionally two recognition sequences for a recombinase flanking the marker gene, two sequences for homologous recombination (homologous arms) flanking the marker and the target gene and optionally at least one marker gene for negative clone selection flanking the homologous arms.

8. The recombinant vector of claim 7, wherein the at least one marker gene for positive clone selection is an antibiotic resistance gene, preferably the gene coding for neomycin resistance and/or the at least one marker gene for negative clone selection is a gene coding for a toxin, preferably for diphteria toxin or a gene coding for thymidine kinase.

9. Murine embryonic stem cells transfected with the recombinant vector of claims 7 or 8.

10. An in-vivo method of screening, investigating and/or preclinical testing of compounds and/or drug candidates for human anti-TNFα therapy comprising the following steps:
a) providing a transgenic mouse of anyone of claims 1 to 5
b) inducing a pathogenic TNF-α response in said mouse
c) treating said mouse of step b) with the compounds and/or drug candidates to be tested
d) analysing the human anti-TNFα potential of said compounds and/or drug candidates administered in step c) by evaluating the changes in animal physiology and/or by comparing the changes in animal physiology with control animals
e) optionally repeating steps c) and d) with an appropriately modified form of the compounds and/or drug candidates to be tested.

11. The method of claim 10, wherein
in step b) LPS-Dgal is injecting into said mouse and/or
in step c) human TNF-α blocking compounds, preferably Infliximab,
Adalimumab, Etanercept, mutant TNFα capable of disrupting existing
TNFα trimers, dominant negative mutants and/or small molecules which
would interfere with TNFα-TNF receptor are injected into said mouse and/or
in step d) the TNF-α plasma levels are analysed and/or the mortality rate of said mice is detected and/or compared to healthy control mice.

12. An in-vitro method of screening, investigating and/or preclinical testing of compounds and/or drug candidates for human anti-TNFα-therapy wherein the cells, tissues and/or cell lines of claim 6 are used.

13. A compound discovered and/or developed using a method according to claims 10 to 12.

14. A method of production of the transgenic mouse of anyone of claims 1 to 5 comprising the steps of
a) generating an appropriate recombinant vector comprising the human TNFα-gene
b) transfecting said recombinant vector of step a) into murine embryonic stem cells;
c) selecting an ES clone with desired mutation in one allele
d) microinjecting an embryonic stem cell of step c) into a murine blastocyst and transferring said blastocyst into a receptor mouse;
e) selecting the offspring until a human TNFα Knock-In mouse is obtained.
